(19)

Europäisches
Patentamt

European
Patent Office

Office européen
des brevets

(11)  **EP 3 289 094 B1**

(12)  # EUROPEAN PATENT SPECIFICATION

(45) Date of publication and mention
of the grant of the patent:
**05.06.2024  Bulletin 2024/23**

(21) Application number: **16787045.0**

(22) Date of filing: **27.04.2016**

(51) International Patent Classification (IPC):
**G01N 33/50** (2006.01)

(52) Cooperative Patent Classification (CPC):
**G01N 33/5011; G01N 33/5079**

(86) International application number:
**PCT/US2016/029510**

(87) International publication number:
**WO 2016/176299 (03.11.2016 Gazette 2016/44)**

(54)  **COMPOSITIONS AND METHODS FOR ASSESSING TOXICITY USING DYNAMIC BH3 PROFILING**

ZUSAMMENSETZUNGEN UND VERFAHREN ZUR BEURTEILUNG VON TOXIZITÄT MITTELS
DYNAMISCHER BH3-PROFILIERUNG

COMPOSITIONS ET MÉTHODES D'ÉVALUATION DE TOXICITÉ AU MOYEN D'UN PROFILAGE
BH3 DYNAMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **27.04.2015  US 201562153475 P**

(43) Date of publication of application:
**07.03.2018  Bulletin 2018/10**

(73) Proprietor: **Dana-Farber Cancer Institute, Inc.
Boston, MA 02215 (US)**

(72) Inventors:
  • **LETAI, Anthony
    Medfield, Massachusetts 02052 (US)**
  • **BHOLA, Patrick
    Cambridge, Massachusetts 02139 (US)**
  • **RYAN, Jeremy
    Somerville, Massachusetts 02145 (US)**

(74) Representative: **Elkington and Fife LLP
Prospect House
8 Pembroke Road
Sevenoaks, Kent TN13 1XR (GB)**

(56) References cited:
WO-A1-2014/047342     WO-A1-2015/010094
WO-A2-03/040168       WO-A2-2013/170176
US-A1- 2010 286 057

• **MONTERO J. ET AL.: "Drug-induced death
signaling strategy rapidly predicts cancer
response to chemotherapy", CELL, vol. 160, no.
5, 26 February 2015 (2015-02-26), pages 977-989,
XP055508174,**
• **LONG, J ET AL.: 'Optimization and Validation of
Mitochondria-based Functional Assay as a
Useful Tool to Identify BH3-like Molecules
Selectively Targeting Anti-apoptotic Bcl-2
Proteins.' BMC BIOTECHNOLOGY. vol. 13, no. 45,
24 May 2013, pages 1 - 10, XP021156538**
• **BURON, N ET AL.: 'Use of Human Cancer Cell
Lines Mitochondria to Explore the Mechanisms
of BH3 Peptides and ABT-737-Induced
Mitochondrial Membrane Permeabilization.'
PLOS ONE. vol. 5, no. 3;, 31 March 2010, pages 1
- 13, XP055326361**

Remarks:
The complete document including Reference
Table(s) and the Sequence Listing(s) can be
downloaded from the EPO website

**Description**

**BACKGROUND**

Dynamic BH3 Profiling is a technique used to measure the sensitivity of cells to a test compound or therapeutic. Dynamic BH3 profiling is applied to a cancer cell sample, and drugs that move the cancer cells in that sample closer to the threshold of programmed cell death can be readily identified. The drugs so identified are those likely to provide clinical anticancer benefit to the subject from which the cancer cell sample was derived, thereby providing personalized therapy for individual cancer patients.

**SUMMARY**

The present disclosure relates to the discovery that the principles of dynamic BH3 profiling can be utilized to assess the toxicity of an agent in non-cancerous cells. In various aspects, the disclosure provides methods for determining how an agent alters the apoptotic sensitivity of non-cancerous cells.

Accordingly, in some aspects, the disclosure provides a method of screening a plurality of agents for determining toxicity, said method comprising: a) providing a plurality of agents; b) providing a test aliquot of a sample of cells for each of the plurality of agents, wherein the sample of cells are non-cancerous cells obtained from a subject; c) separately contacting each test aliquot with its respective agent; d) providing a control aliquot of the sample of cells, wherein the control aliquot is not contacted with any of the plurality of agents; e) permeabilizing the cells in each aliquot; f) contacting the permeabilized cells with a pro-apoptotic BH3 domain peptide; g) determining a value for percent BH3 domain peptide-induced mitochondrial outer membrane permeabilization (MOMP) in the cells in each of the test aliquots; h) determining a value for percent BH3 domain peptide-induced MOMP in the cells in the control aliquot; and i) determining a value for delta percent priming for each of the test aliquots, wherein delta percent priming is the difference between the value for percent MOMP determined in (g) and the value for percent MOMP determined in (h), wherein a delta percent priming of greater than 20 percent indicates that the agent with which the cells in the test aliquot were contacted is toxic to the cells.

In some embodiments, the method further comprises conducting in vivo toxicity testing with the agent in a non-human animal or in vitro toxicity testing with the agent if the delta percent priming is 20 percent or less, 18 percent or less, 16 percent or less, 14 percent or less, 12 percent or less, 10 percent or less, 8 percent or less, 6 percent or less, 4 percent or less, 2 percent or less, or 1 percent or less.

In some embodiments, the non-cancerous cells are healthy cells.

In some embodiments, the method further comprises j) providing a test aliquot of a sample of cancerous cells obtained from a subject for each of the plurality of agents; k) separately contacting each test aliquot with its respective agent; 1) providing a control aliquot of the sample of cancerous cells, wherein the control aliquot is not contacted with any of the plurality of agents; m) permeabilizing the cancerous cells in each aliquot; n) contacting the permeabilized cancerous cells with a pro-apoptotic BH3 domain peptide; o) determining a value for percent BH3 domain peptide-induced MOMP in the cancerous cells in each of the test aliquots; p) determining a value for percent BH3 domain peptide-induced MOMP in the cancerous cells in the control aliquot; and q) determining a value for delta percent priming for each of the test aliquots, wherein delta percent priming is the difference between the value for percent MOMP determined in (o) and the value for percent MOMP determined in (p), wherein a delta percent priming of greater than 20 percent indicates that the agent with which the cancerous cells in the test aliquot were contacted is toxic to the cancerous cells. In some embodiments, the method further comprises conducting in vivo or in vitro toxicity testing with the agent in a non-human animal if the delta percent priming in the cancerous cells is greater than 20 percent, greater than 30 percent, greater than 40 percent, greater than 50 percent, greater than 60 percent, greater than 70 percent, greater than 80 percent, greater than 90 percent, greater than 95 percent, or 100 percent.

In some embodiments, the method further comprises conducting in vivo toxicity testing with the agent in a non-human animal or in vitro toxicity testing with the agent if the delta percent priming in the cancerous cells is greater than the delta percent priming in the non-cancerous cells.

In some embodiments, the method further comprises conducting in vivo toxicity testing with the agent in a non-human animal or in vitro toxicity testing with the agent if the delta percent priming in the cancerous cells is at least 10%, at least 20%, at least 30%, at least 40%, at least 50%, at least 60%, at least 70%, at least 80%, at least 90%, at least 100%, at least 150%, or at least 200%, greater than the delta percent priming in the non-cancerous cells.

In some embodiments, the method further comprises conducting in vivo toxicity testing with the agent in a non-human animal or in vitro toxicity testing with the agent if the delta percent priming in the cancerous cells is at least 2-fold, at least 3-fold, at least 4-fold, at least 5-fold, at least 6-fold, at least 7-fold, at least 8-fold, at least 9-fold, or at least 10-fold greater than the delta percent priming in the non-cancerous cells.

In some embodiments, the cancerous cells are primary human tumor cells. In some embodiments, the cancerous cells are obtained from a patient derived xenograft (PDX).

In some embodiments, the plurality of agents includes 20 or more agents. In some embodiments, the plurality of agents includes 100 or more agents.

In some embodiments, each test aliquot is contacted with its respective agent for 24 hours or less. In some embodiments, the permeabilized cells are contacted with the pro-apoptotic BH3 domain peptide for 3 hours or less.

In some embodiments, the cells in each of the test aliquots are permeabilized with digitonin or saponin.

In some embodiments, the percent BH3 domain peptide-induced MOMP is determined by one or more of: i) contacting the cells with a potentiometric dye and measuring the emission of said potentiometric dye; ii) measuring the release of a molecule from the mitochondrial intermembrane space; and iii) measuring the retention of a molecule from the mitochondrial intermembrane space. In some embodiments, the potentiometric dye is 5,5',6,6'-tetrachloro-1,1',3,3'-tetraethylbenzimidazolylcarbocyanine iodide (JC-1), dihydrorhodamine 123, tetramethylrhodamine methyl ester (TMRM), or tetramethylrhodamine ethyl ester (TMRE). In some embodiments, the molecule from the mitochondrial intermembrane space is cytochrome c, SMAC/Diablo, Omi, adenylate kinase-2, or apoptosis-inducing factor.

In some embodiments, the BH3 domain peptide is derived from the BH3 domain of a BID, a BIM, a BAD, a NOXA, a PUMA, a BMF, or an HRK polypeptide. In some embodiments, the BH3 domain peptide is selected from the group consisting of SEQ ID NO: 1-16.

In some embodiments, an agent from the plurality of agents comprises one or more compounds. In some embodiments, an agent from the plurality of agents is an anticancer agent. In some embodiments, an agent from the plurality of agents is a chemotherapeutic agent. In some embodiments, one or more of the plurality of agents are selected from small organic molecules, small inorganic molecules, peptides, proteins, protein analogs, enzymes, nucleic acids, nucleic acid analogs, antibodies, antigens, hormones, lipids, polysaccharides, growth factors, viruses, cells, bioactive agents, pharmaceutical agents, and combinations and prodrugs thereof. In some embodiments, one or more of the plurality of agents are selected from gases, fine particles, radiation, electromagnetic radiation, and aerosols.

In another aspect, the disclosure provides a method of predicting toxicity of an agent to a tissue, said method comprising: a) providing an agent; b) providing a test aliquot of each of a plurality of cell samples, wherein each cell sample comprises non-cancerous cells obtained from a tissue of a subject; c) separately contacting each test aliquot with the agent; d) providing a control aliquot of each of the plurality of cell samples, wherein the control aliquot is not contacted with the agent; e) permeabilizing the cells in each test aliquot and control aliquot; f) contacting the permeabilized cells with a pro-apoptotic BH3 domain peptide; g) determining a value for percent BH3 domain peptide-induced MOMP in the cells in each test aliquot; h) determining a value for percent BH3 domain peptide-induced MOMP in the cells in each control aliquot; and i) determining a value for delta percent priming for each test aliquot, wherein delta percent priming is the difference between the value for percent MOMP determined in (g) and the value for percent MOMP determined in (h), wherein a delta percent priming of greater than 20 percent indicates that the agent is toxic to the tissue from which the cells were obtained.

In some embodiments, the method further comprises conducting in vivo toxicity testing with the agent in a non-human animal or in vitro toxicity testing with the agent if the delta percent priming is 20 percent or less, 18 percent or less, 16 percent or less, 14 percent or less, 12 percent or less, 10 percent or less, 8 percent or less, 6 percent or less, 4 percent or less, 2 percent or less, or 1 percent or less.

In some embodiments, the non-cancerous cells are healthy cells. In some embodiments, the cells are mammalian cells. In some embodiments, the cells are derived from samples obtained by biopsy or autopsy of an animal.

In some embodiments, the cells in each test aliquot are contacted with the agent for 24 hours or less. In some embodiments, the permeabilized cells are contacted with the pro-apoptotic BH3 domain peptide for 3 hours or less.

In some embodiments, the cells are permeabilized with digitonin or saponin.

In some embodiments, the percent BH3 domain peptide-induced MOMP is determined by one or more of: i) contacting the cells with a potentiometric dye and measuring the emission of said potentiometric dye; ii) measuring the release of a molecule from the mitochondrial intermembrane space; and iii) measuring the retention of a molecule from the mitochondrial intermembrane space. In some embodiments, the potentiometric dye is 5,5',6,6'-tetrachloro-1,1',3,3'-tetraethylbenzimidazolylcarbocyanine iodide (JC-1), dihydrorhodamine 123, tetramethylrhodamine methyl ester (TMRM), or tetramethylrhodamine ethyl ester (TMRE). In some embodiments, the molecule from the mitochondrial intermembrane space is cytochrome c, SMAC/Diablo, Omi, adenylate kinase-2, or apoptosis-inducing factor.

In some embodiments, the BH3 domain peptide is derived from the BH3 domain of a BID, a BIM, a BAD, a NOXA, a PUMA, a BMF, or an HRK polypeptide. In some embodiments, the BH3 domain peptide is selected from the group consisting of SEQ ID NO: 1-16.

In some embodiments, the agent comprises one or more compounds. In some embodiments, the agent is an anticancer agent. In some embodiments, the agent is a chemotherapeutic agent. In some embodiments, the agent is a small organic molecule, small inorganic molecule, peptide, protein, protein analog, enzyme, nucleic acid, nucleic acid analog, antibody, antigen, hormone, lipid, polysaccharide, growth factor, virus, cell, bioactive agent, pharmaceutical agent, or some combination or prodrug thereof. In some embodiments, the agent is gas, fine particles, radiation, electromagnetic radiation, or aerosol.

In another aspect, the disclosure provides a method of predicting toxicity of an agent, said method comprising: a) providing one or more cell samples, wherein each cell sample comprises non-cancerous cells and was obtained from a tissue from a non-human animal that had been contacted with a sublethal dose of an agent and then sacrificed; b) providing a test aliquot of each of the one or more cell samples; c) separately contacting each test aliquot with the agent; d) providing a control aliquot of each of the one or more cell samples, wherein the control aliquot is not contacted with the agent; e) permeabilizing the cells in each aliquot; f) contacting the permeabilized cells with a pro-apoptotic BH3 domain peptide; g) determining a value for percent BH3 domain peptide-induced MOMP in the cells in the test aliquot; h) determining a value for percent BH3 domain peptide-induced MOMP in the cells in the control aliquot; and i) determining a value for delta percent priming for the test aliquot, wherein delta percent priming is the difference between the value for percent MOMP determined in (g) and the value for percent MOMP determined in (h), wherein a delta percent priming of greater than 20 percent indicates that the agent is toxic to the tissue of the non-human animal from which the cells were obtained.

In some embodiments, the non-human animal is a mammal. In some embodiments, the mammal is a non-human primate or a rodent.

In some embodiments, the non-human animal is contacted with the agent by injection, inhalation, topical administration, or oral administration. In some embodiments, the non-human animal is contacted with the agent for 24 hours or less.

In some embodiments, the permeabilized cells are contacted with the pro-apoptotic BH3 domain peptide for 3 hours or less.

In some embodiments, the cells are permeabilized with digitonin or saponin.

In some embodiments, the percent BH3 domain peptide-induced MOMP is determined by one or more of: i) contacting the cells with a potentiometric dye and measuring the emission of said potentiometric dye; ii) measuring the release of a molecule from the mitochondrial intermembrane space; and iii) measuring the retention of a molecule from the mitochondrial intermembrane space. In some embodiments, the potentiometric dye is 5,5',6,6'-tetrachloro-1,1',3,3'-tetraethylbenzimidazolylcarbocyanine iodide (JC-1), dihydrorhodamine 123, tetramethylrhodamine methyl ester (TMRM), or tetramethylrhodamine ethyl ester (TMRE). In some embodiments, the molecule from the mitochondrial intermembrane space is cytochrome c, SMAC/Diablo, Omi, adenylate kinase-2, or apoptosis-inducing factor.

In some embodiments, the BH3 domain peptide is derived from the BH3 domain of a BID, a BIM, a BAD, a NOXA, a PUMA, a BMF, or an HRK polypeptide. In some embodiments, the BH3 domain peptide is selected from the group consisting of SEQ ID NO: 1-16.

In some embodiments, the agent comprises one or more compounds. In some embodiments, the agent is an anticancer agent. In some embodiments, the agent is a chemotherapeutic agent. In some embodiments, the agent is a small organic molecule, small inorganic molecule, peptide, protein, protein analog, enzyme, nucleic acid, nucleic acid analog, antibody, antigen, hormone, lipid, polysaccharide, growth factor, virus, cell, bioactive agent, pharmaceutical agent, or some combination or prodrug thereof. In some embodiments, the agent is gas, fine particles, radiation, electromagnetic radiation, or aerosol.

In some embodiments of any of the provided methods, the cells are cultured on an adhesive solid surface in a culture medium having serum in the presence and absence of an agent. In some embodiments of any of the provided methods, the method further comprises washing the culture media from the cells prior to contacting the cells with the BH3 profiling buffer and the pro-apoptotic BH3 domain peptide.

In some embodiments, the BH3 profiling buffer is added at a concentration of 2X, 3X or 4X. In some embodiments, the BH3 profiling buffer is added at a concentration of 2X and the amount of BH3 domain peptide induced MOMP is measured by microscopy. In some embodiments, the BH3 profiling buffer is added at a concentration of 3X or 4X and the amount of BH3 domain peptide induced MOMP is measured by Fluorescence-activated cell sorting (FACS).

In some embodiments, the adhesive solid surface is coated with one or more pro-adhesive compounds. In some embodiments, the one or more pro-adhesive compounds is an extracellular matrix (ECM) protein. In some embodiments, the ECM protein is selected from the group consisting of collagen 1, laminin, collagen 4 and fibronectin. In some embodiments, the one or more pro-adhesive compounds is an antibody. In some embodiments, the one or more pro-adhesive compounds is streptavidin.

In some embodiments, the BH3 profiling buffer is Derived from Trehalose Experimental Buffer (DTEB) or Mitochondria Experimental Buffer (MEB). In some embodiments, the cells are permeabilized after, prior to, or simultaneously when contacting with the BH3 domain peptide. In some embodiments, the BH3 profiling buffer is supplemented with a permeabilizing agent. In some embodiments, the permeabilizing agent is digitonin or saponin.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

## BRIEF DESCRIPTION OF THE DRAWINGS

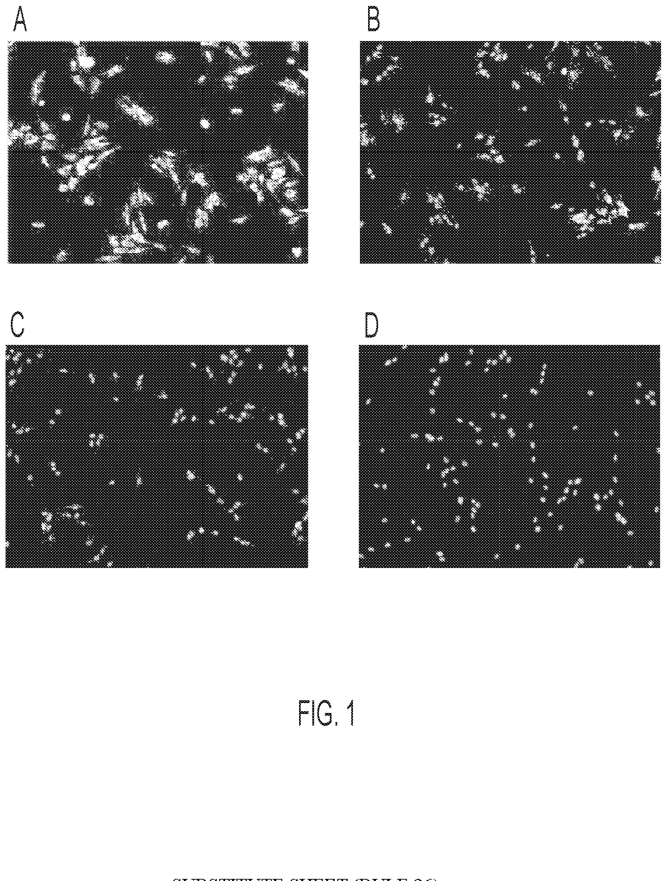

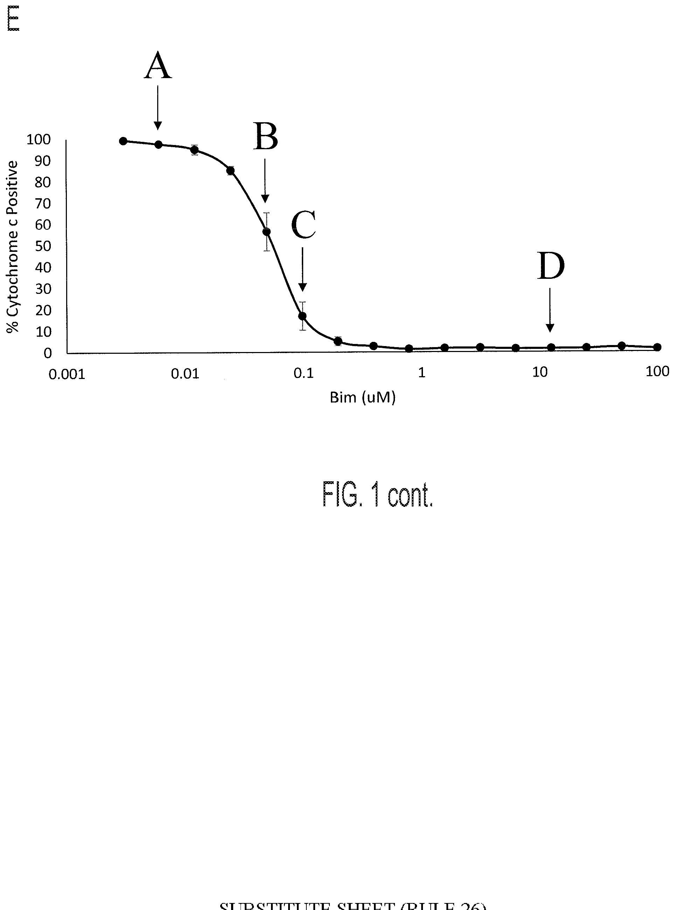

FIGs. 1A-1E depict exemplary BH3 profiling on human foreskin fibroblasts (HFF). To demonstrate BH3 profiling on healthy HFF cells, loss of cytochrome c from mitochondria was measured in response to the synthetic BH3 peptides. Cytochrome c immunofluorescence (FIGs. 1A-1D) was assessed at varied concentrations of the synthetic Bim BH3 peptide along a dose-response curve (FIG. 1E).

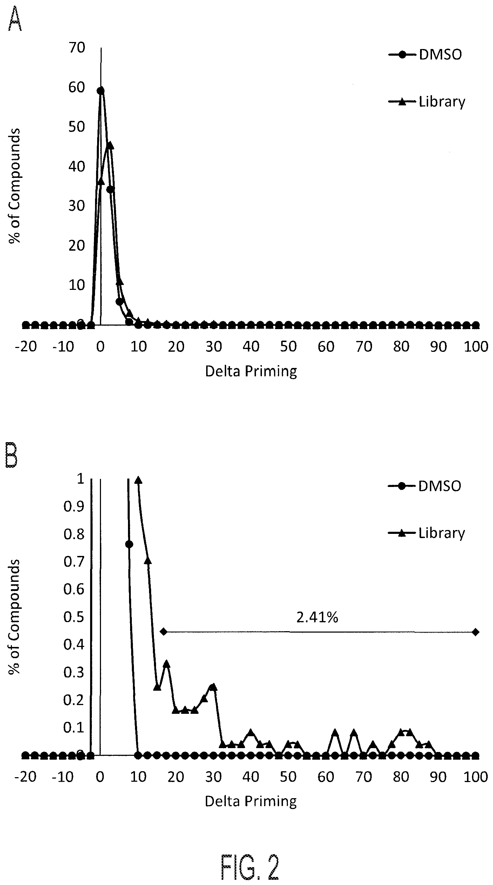

FIGs. 2A-2B depict exemplary drug-induced changes in BH3 profiles in healthy human foreskin fibroblasts. After treating healthy HFFs with a library of compounds or with DMSO (an inactive chemical used as an experimental control) for 20 hours, increases in cell death sensitivity were determined by BH3 profiling. Specifically, a compound that increases apoptotic sensitivity (delta percent priming) is a compound that accelerates loss of cytochrome c. FIG. 2A depicts delta a histograms of the increases in cell death sensitivity (delta percent priming), with FIG. 2B depicting a zoomed view of the y-axis (percentage of compounds in the library) in 2A.

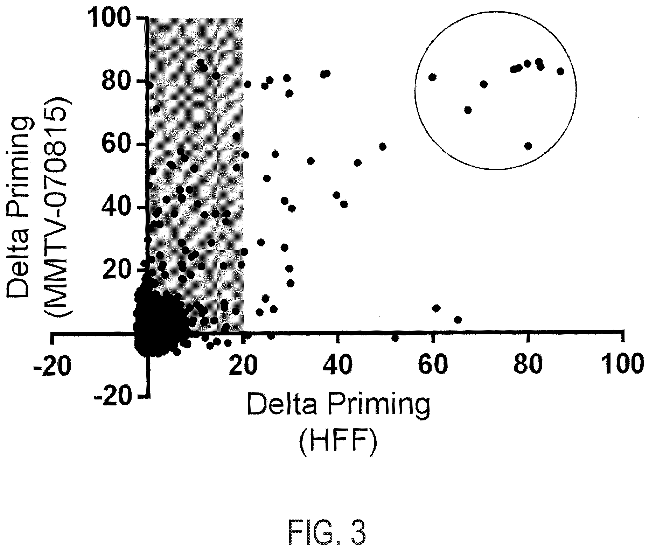

FIG. 3 shows an exemplary comparison of drug induced changes in BH3 profiles between mouse mammary tumor cells (y-axis) and healthy human foreskin fibroblasts. In comparing the effects of ~2400 compounds on cell death sensitivity in the healthy HFF cells or in the tumor cells, several compounds appear to sensitize tumors, but not healthy cells for death (shaded area). Conversely, several compounds appear to sensitize both the healthy HFF cells and the mouse mammary tumor cells for apoptosis (circled area).

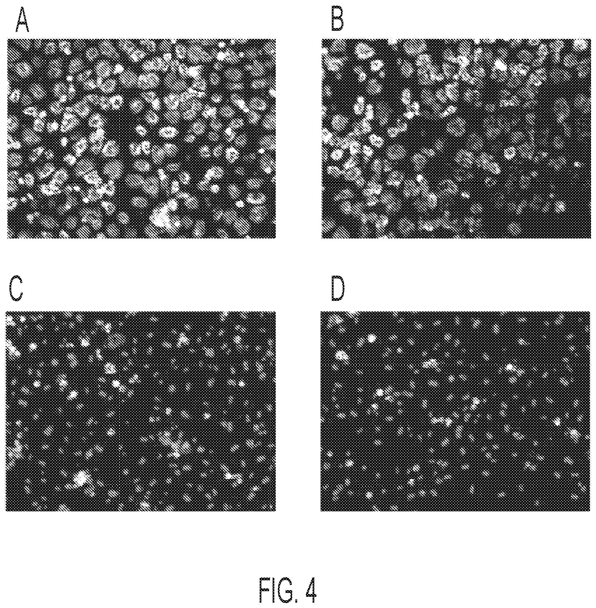

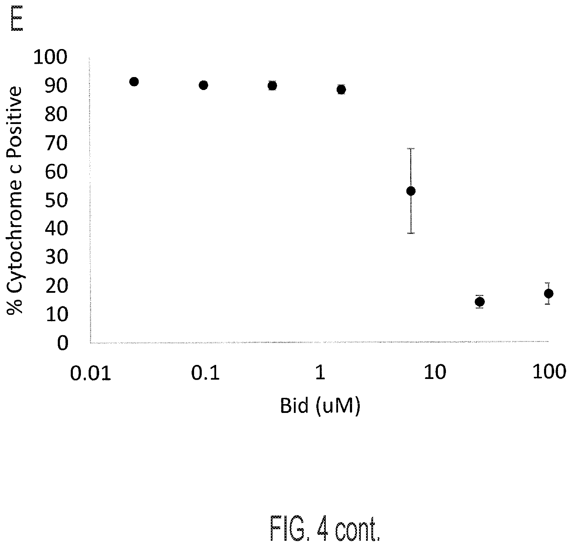

FIGs. 4A-4E depict exemplary BH3 profiling of adult mouse hepatocytes. The loss of cytochrome c from mitochondria was measured in response to the synthetic BH3 peptides. Cytochrome c immunofluorescence was assessed using the synthetic Bim BH3 peptide at 0.1 μM (FIG. 4A), 1.56 μM (FIG. 4B), 25 μM (FIG. 4C), and 100 μM (FIG. 4D). A corresponding dose-response curve is further depicted (FIG. 4E).

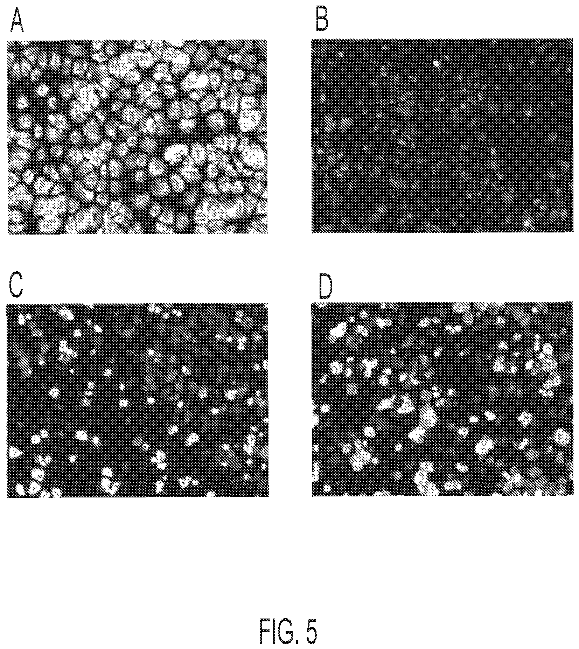

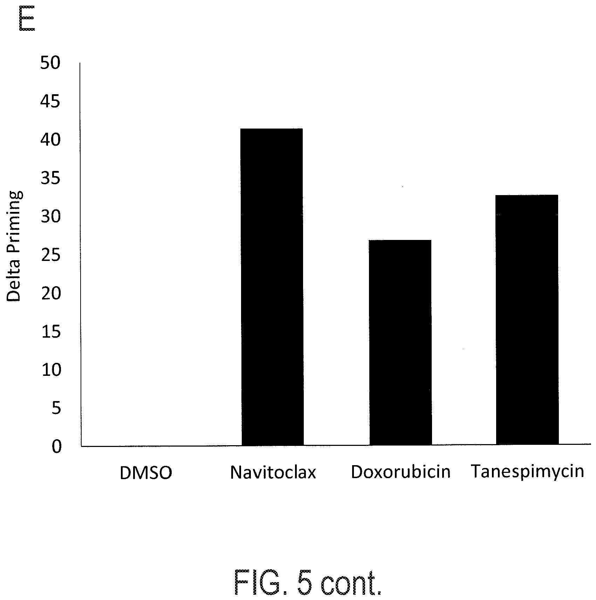

FIGs. 5A-5E depicts examples of compounds that increase cell death sensitivity in adult mouse hepatocytes. The loss of cytochrome c from mitochondria was measured in response to DMSO control (FIG. 5A), Navitoclax (FIG. 5B), Doxorubicin (FIG. 5C), and Tanespimycin (FIG. 5D). Increase in cell death sensitivity is indicated by loss of cytochrome c, which is quantified as an increase in apoptotic sensitivity, or delta priming (*e.g.,* delta percent priming), and is depicted in FIG. 5E.

## DETAILED DESCRIPTION OF THE INVENTION

Dynamic BH3 profiling is a technique used to measure the sensitivity of cells to a test compound or therapeutic. This technique has been described previously in WO 2014/047342 and WO 2015/010094.

The present disclosure relates to the discovery that the principles of dynamic BH3 profiling can be utilized to assess the toxicity of an agent in non-cancerous cells. In various aspects, the disclosure provides methods for quickly and effectively determining how an agent alters the apoptotic sensitivity of non-cancerous cells. For example, many current means of assessing drug toxicity during drug development is limited because pre-clinical toxicity largely relies on testing in mammals, which can be expensive, time consuming, and unreliable. Dynamic BH3 profiling methods provided herein provide inexpensive and robust alternatives to traditional pre-clinical toxicity testing. For example, dynamic BH3 profiling may be carried out on normal cells, *e.g.,* a panel of normal cells, to perform toxicity screens of drugs on normal primary tissues. As another example, many current means of assessing whether a particular environment (*e.g.,* a room, a building, a street, a city) is toxic depend on chromatographic analyses that require prior knowledge of the potential toxin. Dynamic BH3 profiling methods provided herein permit profiling of normal cells to rapidly detect the presence of toxic agents (*e.g.,* radiation, gas, biological agents, *etc.*) in an environment.

In some aspects, the disclosure provides a method of screening a plurality of agents for determining toxicity, including: a) providing a plurality of agents; b) providing a test aliquot of the sample of cells for each of the plurality of agents, wherein the sample of cells are non-cancerous cells obtained from a subject; c) separately contacting each test aliquot with its respective agent; d) providing a control aliquot of the sample of cells, wherein the control aliquot is not contacted with any of the plurality of agents; e) permeabilizing the cells in each aliquot; f) contacting the permeabilized cells with a pro-apoptotic BH3 domain peptide; g) determining a value for percent BH3 domain peptide-induced MOMP in the cells in each of the test aliquots; h) determining a value for percent BH3 domain peptide-induced MOMP in the cells in the control aliquot; and i) determining a value for delta percent priming for each of the test aliquots, wherein delta percent priming is the difference between the value for percent MOMP determined in (g) and the value for percent MOMP determined in (h), wherein a delta percent priming of greater than 20 percent indicates that the agent with which the cells in the test aliquot were contacted is toxic to the cells. In some aspects, the disclosure provides a method of predicting toxicity of an agent to a tissue, said method comprising: a) providing an agent; b) providing a test aliquot of each of a plurality of cell samples, wherein each cell sample comprises non-cancerous cell obtained from a tissue of a subject; c) separately contacting each test aliquot with the agent; d) providing a control aliquot of each of the plurality of cell samples, wherein the control aliquot is not contacted with the agent (*e.g.,* the control aliquot contains cells not contacted with the agent or contains cells from an animal not contacted with the agent); e) permeabilizing the cells in each test aliquot and control aliquot; f) contacting the permeabilized cells with a pro-apoptotic BH3 domain peptide; g) determining a value for percent BH3 domain peptide-induced MOMP in the cells in each test aliquot; h) determining a value for percent BH3 domain peptide-induced MOMP in the cells in each control aliquot; and i) determining a value for delta percent priming for each test aliquot, wherein delta percent priming is the difference between the value for percent MOMP determined in (g) and the value for percent MOMP determined in (h), wherein a delta percent priming of greater than 20 percent indicates that the agent is toxic to the tissue from which the cells were obtained.

In some embodiments, the non-cancerous cells are normal healthy cells (e.g., cells that do not have any patent or latent pathological condition). In some embodiments, the non-cancerous cells are infected with an infectious agent, e.g., a virus or an intracellular bacterium. In some embodiments, the non-cancerous cells are from an organ that is otherwise not functioning normally. In some embodiments, the non-cancerous cells are subject to stress, e.g., vascular stress such as hypoxia, stroke, myocardial infarction, among others. In some embodiments, the cells are mammalian cells. In some embodiments, the cells are derived from samples obtained by biopsy or autopsy of an animal. In some embodiments, the cells are non-malignant human or mouse primary cells.

In some embodiments, provided methods further include j) providing a test aliquot of a sample of cancerous cells obtained from a subject for each of the plurality of agents; k) separately contacting each test aliquot with its respective agent; 1) providing a control aliquot of the sample of cancerous cells, wherein the control aliquot is not contacted with any of the plurality of agents; m) permeabilizing the cancerous cells in each aliquot; n) contacting the permeabilized cancerous cells with a pro-apoptotic BH3 domain peptide; o) determining a value for percent BH3 domain peptide-induced MOMP in the cancerous cells in each of the test aliquots; p) determining a value for percent BH3 domain peptide-induced MOMP in the cancerous cells in the control aliquot; and q) determining a value for delta percent priming for each of the test aliquots, wherein delta percent priming is the difference between the value for percent MOMP determined in (o) and the value for percent MOMP determined in (p), wherein a delta percent priming of greater than 20 percent indicates that the agent with which the cancerous cells in the test aliquot were contacted is toxic to the cancerous cells.

In some embodiments, the method further comprises conducting in vivo toxicity testing with the agent in a non-human animal or in vitro toxicity testing with the agent if the delta percent priming in the cancerous cells is at least 10%, at least 20%, at least 30%, at least 40%, at least 50%, at least 60%, at least 70%, at least 80%, at least 90%, at least 100%, at least 150%, or at least 200%, greater than the delta percent priming in the non-cancerous cells.

In some embodiments, the method further comprises conducting in vivo toxicity testing with the agent in a non-human animal or in vitro toxicity testing with the agent if the delta percent priming in the cancerous cells is at least 2-fold, at least 3-fold, at least 4-fold, at least 5-fold, at least 6-fold, at least 7-fold, at least 8-fold, at least 9-fold, or at least 10-fold greater than the delta percent priming in the non-cancerous cells.

In some embodiments, the cancerous cells comprise an immortalized cancer cell line. In some embodiments, the cells are immortalized mouse or human cancer cell lines. Established cancer cell lines are well-known in the art and include for example pancreatic cancer cell lines (*e.g.,* YAPC, Panc02.03 and SU86.86, *etc.*), breast cancer cell lines (*e.g.,* AU565, BT-20, CAL-120, HMEL and KPL-1, *etc.*), kidney cancer lines (*e.g.,* 769-P, ACNH, HEK TE, SLR 20 and UMRC2, *etc.*), bone cancer cell lines (*e.g.,* CAL-78, HOS, MG-63 and SK-ES-1, *etc.*) and lymphoid cancer cell lines (*e.g.,* AML-193, BDCM, CML-T1 and JM1, *etc.*). The skilled artisan recognizes other cancer cell lines, for example those disclosed in Barretina et al. (The Cancer Cell Line Encyclopedia enables predictive modelling of anticancer drug sensitivity. Nature. 2012 Mar 28;483(7391):603-7. doi: 10.1038/nature11003).

In some embodiments, the cells are derived from a subject. For example, cancer cells may be isolated from a subject by a surgical technique (*e.g.,* biopsy). Thus, in some embodiments, the cells are primary tumor cells. In some embodiments, the cells comprise a patient-derived xenograft (PDX). As used herein, the term "patient-derived xenograft" (PDX) refers to tissue generated by the implantation of cancerous primary tumor into an immunodeficient mouse.

In some embodiments, contacting the cells includes culturing the cells in the presence or absence of an agent. In some embodiments, the cells are cultured in a culture medium under suitable culture conditions in the presence or absence of an agent. A "culture medium" (also referred to herein as a "cell culture medium" or "medium") is a medium for culturing cells containing nutrients that maintain cell viability and support proliferation. The disclosure contemplates various parameters and conditions for culturing the cell. In some embodiments, the culture medium contains serum. The cell culture medium may contain any of the following nutrients in appropriate amounts and combination: salt(s), buffer(s), amino acids, glucose or other sugar(s), antibiotics, serum or serum replacement, and other components such as peptide growth factors, *etc.* Cell culture media are known in the art and may be classified as natural or artificial media. Examples of cell culture media include but are not limited to Minimum Essential Medium (MEM), Dulbecco's Modified Eagle's Medium (DMEM) and Roswell Park Memorial Institute Medium (RPMI). Selection of an appropriate medium for culturing the cell is within the capability of the skilled artisan. Suitable conditions include growing the cell under standard cell culture conditions in a cell culture incubator (*e.g.,* at 37° C in a humidified atmosphere of >80% relative humidity air and 5 to 10% $CO_2$).

In some embodiments, the cells are contacted with an agent, e.g., cultured in the presence of an agent, for at least 30 minutes, 45 minutes, 1 hour, 2 hours, 4 hours, 8 hours, 12 hours, 16 hours, 20 hours, 1 day, at least 2 days, at least 3 days, or at least 4 days. In some embodiments, the cells are contacted with an agent, e.g., cultured in the presence of an agent, for 48 hours or less, 36 hours or less, 24 hours or less, or 12 hours or less.

In some aspects, the disclosure provides a method of predicting toxicity of an agent, said method comprising: a) contacting a non-human animal with a sublethal dose of an agent; b) sacrificing the animal; c) providing one or more cell samples, wherein each cell sample comprises non-cancerous cells and was obtained from a tissue from the animal; d) providing a test aliquot of each of the one or more cell samples; e) separately contacting each test aliquot with the agent; f) providing a control aliquot of each of the one or more cell samples, wherein the control aliquot is not contacted with the agent; g) permeabilizing the cells in each aliquot; h) contacting the permeabilized cells with a pro-apoptotic BH3 domain peptide; i) determining a value for percent BH3 domain peptide-induced MOMP in the cells in the test aliquot; j) determining a value for percent BH3 domain peptide-induced MOMP in the cells in the control aliquot; and k) determining a value for delta percent priming for the test aliquot, wherein delta percent priming is the difference between the value for percent MOMP determined in (i) and the value for percent MOMP determined in (j), wherein a delta percent priming of greater than 20 percent indicates that the agent is toxic to the tissue of the non-human animal from which the cells were obtained.

As used herein, "a subject" is preferably a mammal. The mammal is, for example, a human, non-human primate, mouse, rat, dog, cat, horse, or cow. In some embodiments, the mammal is a non-human primate or a rodent. In some embodiments, the subject is a genetically-modified animal. For example, a mouse can be genetically engineered to develop a particular cancer. In some embodiments, the subject has not been previously diagnosed as having cancer. Alternatively, in some embodiments, the subject has been previously diagnosed as having cancer, and possibly has already undergone treatment for cancer.

As described herein, aspects of the disclosure can relate to contacting a subject (*e.g.,* a non-human animal) with a sublethal dose of an agent. The non-human animal can be contacted with the agent by methods known in the art. In some embodiments, the agent is administered to the animal, *e.g.,* by injection, inhalation, topical administration, or oral administration. For example, the agent can be administered by topical administration (*e.g.,* epicutaneous or inhalational administration), enteral administration (*e.g.,* oral, gastric, or rectal administration), or parenteral administration (*e.g.,* intravenous, intra-arterial, intraosseous, intra-muscular, intracerebral, intracerebroventricular, intrathecal, or subcutaneous administration).

Cell sensitivity to the agent is determined by contacting the cells or cellular component (*e.g.,* mitochondria) with a BH3 profiling buffer and a BH3 domain peptide from the pro-apoptotic BCL-2 family or small molecules with direct mitochondrial activity. This includes, but is not limited to ABT-199, ABT-263, ABT-737, WEHI-539, A-1210477, and ABT-199. The ability of BH3 peptides to induce MOMP is measured in the cells (or cellular component, *e.g.,* mitochondria) exposed to the agent and the control cells (or cellular component, *e.g.,* mitochondria) not exposed to the agent. An increase in BH3 peptide-induced MOMP in the cells cultured in the presence of the agent compared to the amount of BH3 domain peptide induced MOMP in the cells cultured in the absence of the agent indicates that the cells are responsive (*e.g.,* cell death will be induced) to the agent. No change in MOMP in the cells cultured in the presence of the agent compared to the amount of BH3 domain peptide induced MOMP in the cells cultured in the absence of the agent indicates that the drug has no effect on inducing cell death. A decrease in MOMP in the cells cultured in the presence of the agent compared to the amount of BH3 domain peptide induced MOMP in the cells cultured in the absence of the agent indicates that the agent has a desensitizing or protective effect on the cells.

The difference in the level of BH3 domain peptide-induced MOMP of cells that have been exposed to the agent as compared to the level of BH3 domain peptide-induced MOMP of cells that have not been exposed to the agent is statistically significant. By statistically significant, it is meant that the alteration is greater than what might be expected to happen by chance alone. Significant differences may be identified by using an appropriate statistical test. Tests for statistical significance are well known in the art and are exemplified in Applied Statistics for Engineers and Scientists by Petruccelli, Chen and Nandram 1999 Reprint Ed.

As used herein, the term "BH3 Profiling Buffer" refers to an aqueous solution comprising a sugar, a pH buffer, salts, chelators and a carbon source for the electron transport chain that is useful for performing measurements of MOMP.

Pro-apoptotic BCL-2 BH3 proteins and peptides include: Bcl-2 interacting mediator of cell death (BIM); a mutant thereof (BIM AV); BH3 interacting domain death agonist (BID); Bcl-2-associated death promoter (BAD); NOXA; p53 up-regulated modulator of apoptosis (PUMA); Bcl-2-modifying factor (BMF) and harakiri (HRK) (See, Table 1).

In some embodiments, the method comprises permeabilizing the cell after, prior to, or simultaneously when contacting with the BH3 domain peptide. Generally, permeabilization refers to the process of treating a cell with a reagent such that the cell membrane becomes permeable without permeabilizing the mitochondrial outer membrane. Reagents used for permeabilization include organic solvents (*e.g.,* acetone and methanol) and detergents (*e.g.,* digitonin, saponin, Triton X-100 and Tween-20). Without wishing to be bound by any particular theory, the cell is permeabilized to permit the BH3 peptides access to the mitochondria. Cells are permeabilized by methods known in the art. For example, the cell are permeabilized by contacting the cell with digitonin, saponin, methanol, Triton X-100 or other art-recognized cell-permeabilization agents. In some embodiments, the BH3 profiling buffer comprises the permeabilizing reagent, such as digitonin or saponin.

In some embodiments, permeabilized cells are contacted with the pro-apoptotic BH3 domain peptide for 3 hours or less, 2 hours or less, or 1 hour or less.

The skilled artisan recognizes several methods for contacting the cells with the agent, BH3 profiling buffer and/or BH3 domain peptide. For example, automated liquid handling systems are generally utilized for high throughput drug screening. Automated liquid handling systems utilize arrays of liquid dispensing vessels, controlled by a robotic arm, to distribute fixed volumes of liquid to the wells of an assay plate. Generally, the arrays comprise 96, 384 or 1536 liquid dispensing tips. Non-limiting examples of automated liquid handling systems include digital dispensers (*e.g.,* HP D300 Digital Dispenser) and pinning machines (*e.g.,* MULTI-BLOT™ Replicator System, CyBio, Perkin Elmer Janus). Non-automated methods are also contemplated by the disclosure, and include but are not limited to a manual digital repeat multichannel pipette.

After the cells are treated with the BH3 domain peptide, the MOMP is measured. Outer membrane permeabilization can be measured in several ways. For example, outer membrane permeabilization can be measured by determining the loss of mitochondrial membrane potential. Loss of mitochondrial membrane potential is measured by, for example, treating the cells with a potentiometric or radiometric dye. Examples of potentiometric dyes include, but are not limited to, the fluorescent JC-1 probe (5,5',6,6'-tetrachloro-l,l',3,3'-tetraethylbenzimidazolylcarbocyanine iodide) or dihydrorhodamine 123, or tetramethylrhodamine methyl ester (TMRM) or tetramethylrhodamine ethyl ester (TMRE). These and other potentiometric dyes are well-known in the art.

Alternatively, outer membrane permeabilization is determined by measuring the release of a molecule from the mitochondrial intermembrane space or by measuring the retention of a molecule from the mitochondrial intermembrane space. Examples of a molecule from the mitochondrial intermembrane space include cytochrome c, SMAC/Diablo, Omi, adenylate kinase-2, or apoptotic-inducing factor (AIF). The release or retention of a molecule from the mitochondrial intermembrane space can be measured by methods well-known in the art. For example, the release or retention of a molecule can be measured by using an antibody to the molecule, *i.e.,* an antibody to cytochrome c, SMAC/Diablo, Omi, adenylate kinase-2 or apoptotic-inducing factor (AIF). Detection can be for example, by ELISA, FACS, immunoblot, immunofluorescence, immunohistochemistry, plate fluorimetry, fluorescent imaging or automated image analysis. Analysis of the cells can be manually accomplished using a microscope or automated for example by using software such as Cellprofiler to locate nuclei.

Optionally, the cells are fixed prior to measuring outer membrane permeabilization. Cells are fixed by methods known in the art, such as by using an aldehyde (*e.g.,* formaldehyde), or methanol.

Mitochondrial outer membrane permeabilization can be measured at the single cell level or multi-cell level. Additionally, some of the methods disclosed herein allow for subpopulations of cells to be assayed. For example, a tumor-specific or tumor-associated marker (*e.g.,* EpCam) can be used to identify tumor cells. This allows for the ability to discriminate between normal and tumor cells. MOMP is then measured as described herein in the tumor cells.

Advantageously, measurements of MOMP (*e.g.,* measurements of mitochondrial membrane potential, measurements of the release of a molecule from the mitochondrial intermembrane space, measurements of the retention of a molecule from the mitochondrial intermembrane space) can be normalized to facilitate comparative assessments. For example, some aspects of the methods provided herein relate to screening a plurality of agents for determining toxicity by determining BH3 domain peptide-induced MOMP in a cell for each of the plurality of agents. As described herein, BH3 domain peptide-induced MOMP can be measured by methods known in the art. Normalization of measured BH3 peptide-induced MOMP can be performed by methods known in the art, such as described in Ryan, J., Letai, A. (2013) Methods 61(2): 156-164 and Friedman, A., Letai, A., Fisher, D., Flaherty, K. (2015) Nature Reviews Cancer 15: 747-756.

The following is an exemplary, non-limiting, method of normalizing BH3 domain peptide-induced MOMP measurements: A test aliquot of a sample of cells is contacted with an agent. The test aliquot is contacted with a pro-apoptotic BH3 domain peptide. The BH3 domain peptide-induced MOMP in these "treated" cells is measured as described herein and by methods known in the art (*e.g.,* by measuring mitochondrial membrane polarization, by measuring the release of a molecule from the mitochondrial intermembrane space, or by measuring the retention of a molecule from the mitochondrial intermembrane space). A separate aliquot of the sample of cells, a control aliquot, is not contacted with the agent. The control aliquot is contacted with a pro-apoptotic BH3 domain peptide. The BH3 domain peptide-induced MOMP in these "untreated" cells is measured as described herein and by methods known in the art. In some embodiments, the measured MOMP in "treated" and "untreated" cells can be compared by measuring a maximum range for MOMP measurements in a given sample of cells. The maximum range for MOMP measurements can be determined by measuring an "upper limit" value for MOMP and a "lower limit" value for MOMP. For example, an "upper limit" value for MOMP measurements can be obtained by contacting a separate aliquot of the sample of cells with a molecule that is known to be toxic to a cell and measuring a value for MOMP. In some embodiments, the molecule that is known to be toxic to a cell is an uncoupling agent such as carbonyl cyanide-*p*-trifluoromethoxyphenylhydrazone (FCCP) or carbonyl cyanide *m*-chlorophenyl hydrazine (CCCP). A "lower limit" value for MOMP measurements can be obtained by measuring a value for MOMP in a separate aliquot of cells that have not been contacted with an agent, a pro-apoptotic BH3 domain peptide, or a molecule that is known to be toxic to a cell. The value for percent BH3 domain peptide-induced MOMP can be calculated for the test aliquot and the control aliquot using the equation:

$$\%MOMP = \left[ 1 - \left( \frac{(\text{"}Sample\text{"} - \text{"}UpperLimit\text{"})}{(\text{"}LowerLimit\text{"} - \text{"}UpperLimit\text{"})} \right) \right] \times 100,$$

where "%*MOMP*" represents the value for percent BH3 domain peptide-induced MOMP; "*Sample*" represents either the value for BH3 domain peptide-induced MOMP measured in the test aliquot or the value for BH3 domain peptide-induced MOMP measured in the control aliquot; "*UpperLimit*" represents the measured "upper limit" value for MOMP; and "*LowerLimit*" represents the measured "lower limit" value for MOMP.

In some aspects, methods provided herein relate to determining the toxicity of an agent in a sample of cells. In some embodiments, the toxicity of the agent can be determined by determining a value for delta percent priming. As used herein, "delta percent priming" refers to the extent to which an agent shifts a cell closer to the threshold of apoptosis. In some embodiments, normalized values of MOMP (e.g., percent BH3 domain peptide-induced MOMP, or "%*MOMP*") are used to determine a value for delta percent priming. In such embodiments, the value for delta percent priming can be calculated using the equation:

$$Delta\ Percent\ Priming = (\%MOMP_{TEST}) - (\%MOMP_{CONTROL}),$$

where "%*MOMP$_{TEST}$*" represents the value for percent BH3 domain peptide-induced MOMP determined in a test aliquot (cells contacted with the agent); and "%*MOMP$_{CONTROL}$*" represents the value for percent BH3 domain peptide-induced MOMP determined in a control aliquot (cells not contacted with the agent).

Determining a value for delta percent priming can provide an assessment of the toxicity attributable to a particular agent in a given sample of cells. As described herein, the greater the value for delta percent priming of a cell contacted with an agent, the greater the toxicity of the agent to the cell. Thus, in some embodiments, the toxicity of a first agent in a sample of cells can be compared to the toxicity of a second agent in the sample of cells by comparing the value for delta percent priming determined for each agent. Such methods can be useful to screen for agents that are more toxic to a given sample of cells or less toxic to a given sample of cells, depending upon the outcome desired by a practitioner.

In yet other aspects, methods provided herein relate to determining the toxicity of an agent in each of a plurality of cell samples. Thus, in some embodiments, the toxicity of the agent in a first cell sample can be compared to the toxicity of the agent in a second cell sample by comparing the value for delta percent priming determined for the agent in each of the cell samples. Such methods can be useful to assess the toxicity of a given agent across different cell types (*e.g.,* cells obtained from different tissue types).

The disclosure provides methods for determining the toxicity of an agent to a sample of cells, wherein the cells are non-cancerous. In some embodiments, a delta percent priming of greater than 20% indicates that the agent is toxic to the non-cancerous cells. In some embodiments, a delta percent priming of greater than 0% indicates that the agent is toxic to the non-cancerous cells. In some embodiments, methods provided herein further comprise conducting *in vivo* toxicity testing with agent in a non-human animal or *in vitro* toxicity testing with the agent if the delta percent priming in the non-cancerous cells is 20% or less. For example, *in vivo* or *in vitro* toxicity testing is conducted with an agent if the delta percent priming in the non-cancerous cells is 20% or less, 18% or less, 16% or less, 14% or less, 12% or less, 10% or less, 8% or less, 6% or less, 5% or less, 4% or less, 3% or less, 2% or less, 1% or less, 0.9% or less, 0.8% or less, 0.7% or less, 0.6% or less, 0.5% or less, 0.4% or less, 0.3% or less, 0.2% or less, 0.1% or less, or 0%.

As described herein, aspects of the disclosure provide methods relating to determining toxicity of an agent in a sample of non-cancerous cells. In some embodiments, methods provided herein can further comprise steps relating to determining toxicity of the agent in a sample of cancerous cells. In some embodiments, the toxicity of the agent in the cancerous cells can be assessed using methods provided herein, for example, by determining a value for delta percent priming. In some embodiments, a delta percent priming of greater than 20% indicates that the agent is toxic to the cancerous cells. In some embodiments, a delta percent priming of greater than 0% indicates that the agent is toxic to the cancerous cells. In some embodiments, methods provided herein further comprise conducting *in vivo* toxicity testing with the agent in a non-human animal or *in vitro* toxicity testing with the agent if the delta percent priming in the cancerous cells is greater than 20%. For example, *in vivo* toxicity testing with the agent in a non-human animal or *in vitro* toxicity testing is conducted with an agent if the delta percent priming in the cancerous cells is greater than 20%, greater than 25%, greater than 30%, greater than 35%, greater than 40%, greater than 45%, greater than 50%, greater than 55%, greater than 60%, greater than 65%, greater than 70%, greater than 75%, greater than 80%, greater than 85%, greater than 90%, greater than 95%, greater than 98%, greater than 99%, or 100%. In some embodiments, methods provided herein further comprise conducting *in vivo* toxicity testing with the agent in a non-human animal or *in vitro* toxicity testing with the agent if the delta percent priming in the non-cancerous cells is lower than the delta percent priming in the cancerous cells.

It should be appreciated that *in vivo* or *in vitro* toxicity testing can comprise methods provided herein and methods known in the art related to determining the toxicity of an agent. For example, certain aspects of the disclosure relate to the screening of a plurality of agents for determining toxicity in a sample of non-cancerous cells. In some embodiments, if an agent is not determined to be toxic to the non-cancerous cells, the agent can be selected for further toxicity testing (*e.g., in vivo* or *in vitro* toxicity testing). In such embodiments, it can be useful to conduct further testing comprising determining the toxicity of the agent in a different sample of cells (*e.g.,* cancerous cells, cells obtained from a different type of tissue, cells obtained from a different subject). In some embodiments, it can be useful to conduct further testing comprising determining the toxicity of the agent in a subject (*e.g.,* by administering the agent to the subject, by contacting the agent with a sample of cells obtained from the subject).

A person of skill in the art would appreciate that aspects of the present disclosure can be useful in the optimization of an agent for therapeutic purposes. For example, in some embodiments, methods of determining the toxicity of an agent to both cancerous cells and non-cancerous cells are provided herein. It should be appreciated that, in some embodiments, it is advantageous for an agent (*e.g.,* a therapeutic agent, a cancer therapeutic) to have minimal toxicity in non-cancerous (*e.g.,* healthy) cells and maximal toxicity in cancerous cells. As described herein, such an agent would manifest as having a minimal value for delta percent priming in non-cancerous cells and a maximal value for delta percent priming in cancerous cells.

In some aspects, the disclosure relates to drug discovery. In some embodiments, methods described by the disclosure are useful for screening large libraries of candidate agents to identify new drugs that do not move cells closer to programmed cell death, e.g., agents that are not toxic to the cells. In some embodiments, the library of candidate agents includes 20 or more, 30 or more, 40 or more, 50 or more, 60 or more, 70 or more, 80 or more, 90 or more, 100 or more, 150 or more, 200 or more, 250 or more, or 300 or more agents.

In some embodiments, an agent includes a compound. In some embodiments, an agent includes more than one compound. Exemplary candidate agents include, but are not limited to small organic molecules, small inorganic molecules, peptides, proteins, protein analogs, enzymes, nucleic acids, nucleic acid analogs, antibodies, antigens, hormones, lipids, polysaccharides, growth factors, viruses, cells, bioactive agents, pharmaceutical agents, and combinations and prodrugs thereof. In some embodiments, an agent is an anticancer agent, such as, a chemotherapeutic agent. Further exemplary candidate agents include, but are not limited to, gases, fine particles, radiation, electromagnetic radiation, and aerosols.

In some embodiments, a candidate agent is a chemotherapeutic agent. Non-limiting examples of chemotherapeutic agents include small molecules, peptides or proteins (*e.g.,* peptide antibiotics and antibodies) and RNA-interference (RNAi) molecules. Examples of small molecule chemotherapeutic agents include alkylating agents (cyclophosphamide, chlormethine, temozolomide), anthracyclines (daunorubicin, doxorubicin, mitoxantrone), taxanes (paclitaxel, docetaxel), histone deacetylase inhibitors (vorinostat, romidepsin), topoisomerase I/II inhibitors (irinotecan, topotecan, etoposide), kinase inhibitors (gefitinib, imatinib, bortezomib), nucleotide analogs and precursor analogs (azacitidine, fluorouracil, methotrexate), platinum-based agents (cisplatin, carboplatin), retinoids (alitretinoin, bexarotene) and vinca alkaloids (vinblastine, vindesine, vinorelbine). Examples of peptides and proteins include bleomycin, dactinomycin, antitumor antibodies (anti-HER2/neu, alemtuzumab, trastuzumab, brentuximab). The skilled artisan recognizes chemotherapeutic RNAi molecules as RNAi molecules that target expression of genes related to cancer. For example, RNAi molecules directed against *Hox*A1 can inhibit mammary tumor cell formation, as disclosed by Brock et al. Sci Transl Med 6: 217ra2 (2014). In some embodiments, chemotherapeutic agents include, but are not limited to, kinase inhibitors, apoptosis inducers, angiogenesis inhibitors, and monoclonal antibodies.

In some embodiments, methods provided herein can be useful for determining the toxicity of an environment or environmental condition. For example, in some embodiments, the environment comprises an agent that may or may not be toxic to a cell. In some embodiments, agents in an environment or environmental condition can include, without limitation, gases, fine particles, radiation, electromagnetic radiation, and aerosols. Environmental agents are known in the art, for example, as provided by the National Institute of Environmental Health Sciences (www.niehs.nih.gov/health/topics/agents/). In such embodiments, it should be appreciated that cells can be contacted with the agent using various methods known in the art and described herein. For example, a cell can be exposed to an environment that is suspected of being radioactive or known to be radioactive. In such an embodiment, the agent being tested for toxicity could comprise radiation (*e.g.,* alpha particles, beta particles, positron particles, gamma rays, X-rays, and neutrons).

In some aspects, the disclosure relates to personalized medicine. In some embodiments, methods described herein are useful for the customization of chemotherapeutic regimens. For example, methods provided herein can relate to determining the toxicity of an agent in both non-cancerous cells and cancerous cells. In some embodiments, the non-cancerous cells and the cancerous cells are obtained from a subject. By determining the toxicity of the agent in non-cancerous cells (*e.g.,* normal cells, healthy cells) from the subject and determining the toxicity of the agent in cancerous cells from the subject, the agent can be identified as being more or less appropriate for treating the subject. As described herein, the agent becomes more appropriate for treating the subject as the ratio of delta percent priming in cancerous cells to delta percent priming in non-cancerous cells increases. For example, in determining an appropriate agent for treating a subject who is known to have or is suspected of having cancer, it is preferable to use an agent that has minimal toxicity (*e.g.,* minimal value for delta percent priming) in non-cancerous cells of the patient and maximal toxicity (*e.g.,* maximal value for delta percent priming) in cancerous cells of the patient. Such an agent would selectively target the cells intended for treatment while minimizing negative peripheral effects.

In some aspects, the disclosure provides methods of determining the toxicity of an agent, wherein the toxicity of the agent is determined by comparing BH3 domain peptide-induced MOMP in test cells contacted with the agent to BH3 domain peptide-induced MOMP to control cells not contacted with the agent. In some embodiments, the control cells have not been contacted with any agent. In some embodiments, the control cells have been contacted with buffer or solvent, e.g., buffer or solvent used as a vehicle for the agent. For example, the test cells can be contacted with an agent that has been dissolved in a suitable buffer or solvent (*e.g.,* DMSO), and the control can be contacted with a suitable buffer or solvent (*e.g.,* DMSO) lacking the agent.

In some embodiments, methods described herein can be suitable for a high-throughput format. For example, aspects of the disclosure relate to methods of screening of a plurality of agents to determine toxicity. In such methods, it can be desirable to utilize a multi-well plate. In some embodiments, the plate comprises an agent. In some embodiments, the plate comprises a plurality of agents. For example, the plate may be used to test a panel of agents, each well comprising an agent. In some embodiments, one or more wells of the plate do not comprise an agent. For example, as described herein, control aliquots are not contacted with an agent. In some embodiments, one or more wells designated for control aliquots can comprise buffer or solvent (*e.g.,* DMSO). In some embodiments, the multi-well plate comprises a BH3 domain peptide. In some embodiments, the BH3 domain peptide is derived from the BH3 domain of a BID, a BIM, a BAD, a NOXA, a PUMA a BMF, or a HRK polypeptide. In some embodiments, the BH3 domain peptide is selected from the group consisting of SEQ ID NO: 1-15. In some embodiments, the plate comprises more than one BH3 domain peptide. For example, in some embodiments, a plate comprises at least 2, at least 3, at least 4, at least 5, at least 6, at least 7, at least 8, at least 9 or at least 10 BH3 domain peptides. In some embodiments, one or more wells of the plate do not comprise a BH3 domain peptide. For example, as described herein, it may be desirable to establish a lower limit for MOMP measurements. In such embodiments, aliquots are not contacted with a BH3 domain peptide. In some embodiments, one or more wells designated for lower limit measurements can comprise buffer or solvent (*e.g.,* DMSO).

It will be appreciated that cells do not need to be removed from a culture plate to perform dynamic BH3 profiling described herein. The presence of cell culture media and serum has been demonstrated not to interfere with the ability of BH3 peptides to induce mitochondrial outer membrane permeabilization. Furthermore, wash steps where there is little residual volume above the cells have been demonstrated to be unnecessary. Instead, the cells can be successively treated in the plate well with the drug and the BH3 domain peptide. In some embodiments of any of the provided methods, the cells are cultured on an adhesive solid surface in a culture medium having serum in the presence and absence of an agent. In some embodiments of any of the provided methods, the method further comprises washing the culture media from the cells prior to contacting the cells with the BH3 profiling buffer and the pro-apoptotic BH3 domain peptide.

In some embodiments of any of the provided methods, the adhesive solid surface is coated with one or more pro-adhesive compounds. In some embodiments of any of the provided methods, the one or more pro-adhesive compounds is an extracellular matrix (ECM) protein. In some embodiments of any of the provided methods, the ECM protein is selected from the group consisting of collagen 1, laminin, collagen 4 and fibronectin. In some embodiments, the one or more pro-adhesive compounds is an antibody. In some embodiments, the one or more pro-adhesive compounds is streptavidin.

In some embodiments of any of the provided methods, the BH3 profiling buffer is Derived from Trehalose Experimental Buffer (DTEB) or Mitochondria Experimental Buffer (MEB). In some embodiments of any of the provided methods, the cells are permeabilized after, prior to, or simultaneously when contacting with the BH3 domain peptide. In some embodiments, the BH3 profiling buffer is supplemented with a permeabilizing agent. In some embodiments, the permeabilizing agent is digitonin or saponin

As used here, the term "BH3 Profiling Buffer" refers to an aqueous solution comprising a sugar, a pH buffer, salts, chelators and a carbon source for the electron transport chain that is useful for performing measurements of MOMP. In some embodiments of any of the provided methods, the BH3 Profiling Buffer is a Derived from Trehalose Experimental Buffer (DTEB). In some embodiments, the BH3 Profiling Buffer is a Mitochondria Experimental Buffer (MEB). DTEB is comprised of 135 mM trehalose, 10 mM Hepes, 50 mM KCl, 20 µM EGTA, 20 µM EDTA, 0.1 % BSA and 5mM Succinate. MEB is comprised of 150 mM mannitol, 10 mM Hepes, 50 mM KCl, 20 µM EGTA, 20 µM EDTA, 0.1 % BSA and 5mM Succinate. Sucrose and other sugars may be used in the place of mannitol. Some increases in KCl are tolerated, however can be detrimental to BH3 profiling. Concentrated buffers (2X-5X) involve proportionally increasing the concentration of the above reagents

In some embodiments of any of the provided methods, the BH3 profiling buffer is added at a concentration of 2X, 3X or 4X. In some embodiments of any of the provided methods, the BH3 profiling buffer is added at a concentration of 2X and the amount of BH3 domain peptide induced MOMP is measured by microscopy. In some embodiments of any of the provided methods, the BH3 profiling buffer is added at a concentration of 3X or 4X and the amount of BH3 domain peptide induced MOMP is measured by Fluorescence-activated cell sorting (FACS).

The skilled artisan recognizes several methods for adding the agent, BH3 profiling buffer and/or BH3 domain peptide to the cultured cells. For example, automated liquid handling systems are generally utilized for high throughput drug screening. Automated liquid handling systems utilize arrays of liquid dispensing vessels, controlled by a robotic arm, to distribute fixed volumes of liquid to the wells of an assay plate. Generally, the arrays comprise 96, 384 or 1536 liquid dispensing tips. Non-limiting examples of automated liquid handling systems include digital dispensers (e.g., HP D300 Digital Dispenser) and pinning machines (e.g., MULTI-BLOT™ Replicator System, CyBio, Perkin Elmer Janus). Non-automated methods are also contemplated by the disclosure, and include but are not limited to a manual digital repeat multichannel pipette.

*Pro-Apoptotic BCL-2 BH3 Domain Peptides*

Pro-Apoptotic BCL-2 BH3 domain peptides have been described previously in WO 2014/047342. In particular, a Pro-apoptotic BCL-2 BH3 domain peptide is less than 195 amino acids in length, *e.g.,* less than or equal to 150, 100, 75, 50, 35, 25 or 15 amino acid in length. Non-limiting examples of pro-apoptotic BCL-2 BH3 peptides include: Bcl-2 interacting mediator of cell death (BIM); a mutant thereof (BIM AV); BH3 interacting domain death agonist (BID); Bcl-2-associated death promoter (BAD); NOXA; p53 up-regulated modulator of apoptosis (PUMA); Bcl-2-modifying factor (BMF) and harakiri (HRK).

In some embodiments, a pro-apoptotic BCL-2 BH3 domain peptide includes the sequence of SEQ ID NO: 1-15 shown in Table 1. PUMA2A (SEQ ID NO: 16) is a negative control peptide.

Table 1: Pro-Apoptotic

| Peptide | Sequence | SEQ ID NO: |
|---|---|---|
| BIM | Ac-MRPEIWIAQELRRIGDEFNA-NH2 | 1 |
| BIM AC | AC-MRPEIWIAQELRRIGDEFNV-NH2 | 2 |
| BID | EDIIRNIARBLAQ VGD SMDR | 3 |
| BIM AV | MRPEIWIAQELRRIGDEFNA | 4 |
| BID mut | EDIIRNIARHAAQVGASMDR | 5 |
| BAD | LWAAQRYGRELRRMSDEFEGSFKGL | 6 |
| BIK | MEGSDALALRLACIGDEMDV | 7 |
| NOXA A | AELPPEFAAQLRKIGDKVYC | 8 |
| NOXA B | PADLKDECAQLRRIGDKVNL | 9 |
| HRK S S | AAQLTAARLKALGDELHQ | 10 |
| PUMA | EQWAREIGAQLRRMADDLNA | 11 |
| BMF | HQAEVQIARKLQLIADQFHR | 12 |
| huBAD | NLWAAQRYGRELRRMSDEFVDSFKK | 13 |
| BAD mut | LWAAQRYGREARRMSDEFEGSFKGL | 14 |
| MS1 | RPEIWMTQGLRRLGDEINAYYAR | 15 |
| PUMA2A | EQWAREIGAQARRMAADLNA | 16 |

In some embodiments, a BH3 domain peptide include a peptide which includes (in whole or in part) the sequence

NH$_2$-XXXXXXXXXXLXXXXDXXXX -COOH (SEQ ID NO: 17). As used herein X may be any amino acid. Alternatively, the BH3 domain peptides include at least 5, 6, 7, 8, 9, 15 or more amino acids of SEQ ID NO: 17.

The BH3 domain peptides can be modified using standard modifications. Modifications may occur at the amino (N-), carboxy (C-) terminus, internally or a combination of any of the preceding. In one aspect described herein, there may be more than one type of modification on the polypeptide. Modifications include but are not limited to: acetylation, amidation, biotinylation, cinnamoylation, farnesylation, formylation, myristoylation, palmitoylation, phosphorylation (Ser, Tyr or Thr), stearoylation, succinylation, sulfurylation and cyclisation (via disulfide bridges or amide cyclisation), and modification by Cys3 or Cys5. The modified BH3 domain peptides retain the biological activity of BH3 domain peptides. By retaining the biological activity, it is meant that cell death is induced by the BH3 polypeptide, although not necessarily at the same level of potency as that of the naturally-occurring BH3 domain polypeptide. The terms induced and stimulated are used interchangeably throughout the specification.

Optionally, the BH3 domain peptide is attached to a transduction domain. A transduction domain directs a peptide in which it is present to a desired cellular destination. Thus, the transduction domain can direct the peptide across the plasma membrane, e.g., from outside the cell, through the plasma membrane, and into the cytoplasm. Alternatively, or in addition, the transduction domain can direct the peptide to a desired location within the cell, *e.g.,* the nucleus, the ribosome, the ER, mitochondria, a lysosome, or peroxisome. In some embodiments, the transduction domain is derived from a known membrane-translocating sequence. Alternatively, transduction domain is a compound that is known to facilitate membrane uptake such as polyethylene glycol, cholesterol moieties, octanoic acid and decanoic acid. The transduction domain may be linked either to the N-terminal or the C-terminal end of BH3 domain peptide.

The BH3 domain peptides and/or the transduction domain peptides can be polymers of L-amino acids, D-amino acids, or a combination of both. Alternatively, the BH3 domain peptides and/or the transduction domain peptides are cyclic peptides. Cyclic peptides are prepared by methods known in the art. For example, macrocyclization is often accomplished by forming an amide bond between the peptide N- and C-termini, between a side chain and the N- or C-terminus [e.g., with K3Fe(CN)6 at pH 8.5] (Samson et al., Endocrinology, 137: 5182-5185 (1996)), or between two amino acid side chains. See, *e.g.,* DeGrado, Adv Protein Chem, 39: 51-124 (1988).

BH3 domain peptides and/or the transduction domain peptides are prepared using modern cloning techniques, or may be synthesized by solid state methods or by site-directed mutagenesis. In some embodiments, native BH3 domain peptides and/or transduction domain peptides can be isolated from cells or tissue sources by an appropriate purification scheme using standard protein purification techniques. In another embodiment, BH3 domain polypeptides and/or transduction domain peptides are produced by recombinant DNA techniques. Alternative to recombinant expression, BH3 domain peptides and/or transduction domain peptides can be synthesized chemically using standard peptide synthesis techniques.

In various embodiments, the BH3 peptide maintains its secondary structure, *e.g.,* $\alpha$-helical structure. Methods of helix stabilization are known in the art.

An "isolated" or "purified" BH3 domain peptide is substantially free of cellular material or other contaminating proteins from the cell or tissue source from which the BH3 domain peptide is derived, or substantially free from chemical precursors or other chemicals when chemically synthesized.

*Kits*

The present disclosure also includes kits for performing BH3 Profiling. The kit may comprise a multi-well plate having an agent and a BH3 domain peptide. The kit may comprise a multi-well plate having an agent. The agent may be a chemotherapeutic agent. The kit may comprise a multi-well plate having at least 2, at least 3, at least 4, at least 5, at least 6, at least 7, at least 8, at least 9 or at least 10 agents. The kit may comprise a multi-well plate, wherein each well of the plate comprises a different agent. The kit may comprise a multi-well plate having a BH3 domain peptide. The BH3 domain peptide may be derived from the BH3 domain of a BID, a BIM, a BAD, a NOXA, a PUMA BMF, or a HRK polypeptide. The BH3 domain peptide may be selected from the group consisting of SEQ ID NO: 1-15.

The kit may further comprise a vial containing a BH3 profiling buffer. In some embodiments, the vial is a glass or plastic vial. The vial may comprise volumetric markings on its surface. The BH3 profiling buffer may be supplemented with a permeabilizing agent. The permeabilizing agent may be digitonin or saponin.

The kit may further comprise a potentiometric dye. The potentiometric dye may be 5,5',6,6'- tetrachloro-l,l',3,3'- tetraethylbenzimidazolylcarbocyanine iodide (JC-1), dihydrorhodamine 123, tetramethylrhodamine methyl ester (TMRM) or tetramethylrhodamine ethyl ester (TMRE). Alternatively, the kit may further comprise an antibody for cytochrome c, SMAC/Diablo, Omi, adenylate kinase-2 or apoptosis-inducing factor.

tThe kit may further comprise instructions for using the kit to predict the sensitivity of cells to an agent. Instructions are generally provided as a printed pamphlet or paper sheet but may also include any oral or electronic instructions provided in any manner such that a user will clearly recognize that the instructions are to be associated with the kit, for example, audiovisual (*e.g.,* videotape, DVD, etc.), Internet, and/or web-based communications, etc.

The invention will be further illustrated in the following non-limiting examples.

## EXAMPLES

Example 1: Predicting toxicity in normal cells with dynamic BH3 profiling

There is a great need to predict toxicity of agents to normal tissues. This is most apparent in the case of drug development. Pre-clinical toxicity largely relies on testing in mammals which is expensive and can be unreliable. Testing using dynamic BH3 profiling on normal tissues derived from animals, including human tissues, could alleviate concerns regarding expense and reliability. The concept includes measurement of the provocation of death signaling at short time points after exposure of normal cells to the agent of interest. Using an array of normal cells, measurement of death signaling may be interpreted as being a flag for potential toxicity.

To demonstrate BH3 profiling on healthy human foreskin fibroblasts (HFF) cells, loss of cytochrome c from mitochondria was measured in response to the synthetic BH3 peptides. To do this, cytochrome c immunofluorescence (FIGs. 1A-D) was assessed at increasing concentrations of the synthetic Bim BH3 peptide. An exemplary dose-response curve of cytochrome c loss for increasing concentrations of the synthetic Bim BH3 peptide is depicted in FIG. 1E (standard deviations depicted as error bars). Note that at low concentrations of the Bim peptide there is little or no loss of cytochrome c, whereas at high peptide concentrations a complete loss of cytochrome c is observed.

Next, drug-induced changes in BH3 profiles in healthy human foreskin fibroblasts was examined. After treating healthy HFFs with a library of ~2400 different drug compounds or with DMSO (an inactive chemical used as an experimental control) for 20 hours, increases in cell death sensitivity were determined by BH3 profiling. Specifically, a compound that increases apoptotic sensitivity (delta priming) is a compound that accelerates loss of cytochrome c (FIG. 2). FIG. 2A depicts an exemplary histogram of the increases in cell death sensitivity (delta priming) represented by values of delta percent priming, with FIG 2B representing a zoomed view of the y-axis of the histogram (percentage of compounds in the library). Note that the drug treated healthy HFF cells show an increase in cell death sensitivity compared to the healthy cells treated with DMSO (the inactive chemical) with approximately 2.41% of compounds tested. This indicates that molecules that increase cell death sensitivity in healthy cells relative to a control compound that has no effect can be reliably detected.

The experiments exemplified in FIG. 2 were repeated using mouse mammary tumor cells. A comparison of drug induced changes in BH3 profiles between mouse mammary tumor cells (y-axis) and healthy human foreskin fibroblasts (x-axis) is depicted in FIG. 3. When comparing the effects of ~2400 compounds on increased cell death sensitivity, it was found that there are several compounds that sensitize tumors, but not healthy cells for death (shaded area). Conversely, several compounds appear to sensitize both the healthy HFF cells and the mouse mammary tumor cells for apoptosis. These latter compounds are those that likely have generally toxic effects to both tumor and healthy cells, and have little therapeutic value. However, the former compounds (shaded area) are those that would preferentially target apoptosis in tumor cells over healthy cells as delta priming increases along the y-axis.

BH3 profiling on healthy adult mouse hepatocytes was examined by measuring the loss of cytochrome c from mitochondria in response to the synthetic BH3 peptides. To do this, immunofluorescence for cytochrome c was assessed at increasing concentrations of Bim BH3 peptide (0.1 $\mu$M (FIG. 4A), 1.56 $\mu$M (FIG. 4B), 25 $\mu$M (FIG. 4C), and 100 $\mu$M (FIG. 4D)). A dose-response curve of cytochrome c loss is depicted in FIG. 4E. Note that at low concentrations of the Bim peptide, there is little or no loss of cytochrome c, whereas at high peptide concentrations a complete loss of cytochrome c is observed.

Next, various drug compounds were tested to assess the extent of drug-induced increased cell death sensitivity in adult mouse hepatocytes. Freshly isolated adult mouse hepatocytes were treated with different drugs for 20 hours, and the loss of cytochrome c induced by a single concentration of the synthetic Bim BH3 peptide was subsequently assessed by immunofluorescence (FIGs. 5A-D). Increase in cell death sensitivity is indicated by loss of cytochrome c, which is quantified as an increase in apoptotic sensitivity (delta priming, FIG. 5E). Navitoclax, doxorubicin, and tanespimycin all increase apoptotic sensitivity.

## OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Claims**

1. A method of screening a plurality of agents for determining toxicity, said method comprising:

    a) providing a plurality of agents;
    b) providing a test aliquot of a sample of cells for each of the plurality of agents, wherein the sample of cells are non-cancerous cells previously obtained from a subject:
    c) separately contacting each test aliquot with its respective agent;
    d) providing a control aliquot of the sample of cells, wherein the control aliquot is not contacted with any of the plurality of agents;
    e) permeabilizing the cells in each aliquot;
    f) contacting the permeabilized cells with a pro-apoptotic Bcl-2 homology 3 (BH3) domain peptide;
    g) determining a value for percent BH3 domain peptide-induced mitochondrial outer membrane permeabilization (MOMP) in the cells in each of the test aliquots;
    h) determining a value for percent BH3 domain peptide-induced MOMP in the cells in the control aliquot; and
    i) determining a value for delta percent priming for each of the test aliquots, wherein delta percent priming is the difference between the value for percent MOMP determined in (g) and the value for percent MOMP determined in (h),
    wherein a delta percent priming of greater than 20 percent indicates that the agent with which the cells in the test aliquot were contacted is toxic to the cells.

2. The method of claim 1, further comprising conducting *in vivo* toxicity testing with the agent in a non-human animal or *in vitro* toxicity testing with the agent if the delta percent priming is 20 percent or less, 18 percent or less, 16 percent or less, 14 percent or less, 12 percent or less, 10 percent or less, 8 percent or less, 6 percent or less, 4 percent or less, 2 percent or less, or 1 percent or less; and/or wherein the non-cancerous cells are healthy cells.

3. The method of any one of claims 1-2, further comprising:

    j) providing a test aliquot of a sample of cancerous cells previously obtained from a subject for each of the plurality of agents;
    k) separately contacting each test aliquot with its respective agent;
    l) providing a control aliquot of the sample of cancerous cells, wherein the control aliquot is not contacted with any of the plurality of agents;
    m) permeabilizing the cancerous cells in each aliquot;
    n) contacting the permeabilized cancerous cells with a pro-apoptotic BH3 domain peptide;
    o) determining a value for percent BH3 domain peptide-induced MOMP in the cancerous cells in each of the test aliquots;
    p) determining a value for percent BH3 domain peptide-induced MOMP in the cancerous cells in the control aliquot; and
    q) determining a value for delta percent priming for each of the test aliquots, wherein delta percent priming is the difference between the value for percent MOMP determined in (o) and the value for percent MOMP determined in (p),

    wherein a delta percent priming of greater than 20 percent indicates that the agent with which the cancerous cells in the test aliquot were contacted is toxic to the cancerous cells.

4. The method of claim 3, further comprising conducting *in vivo* toxicity testing with the agent in a non-human animal or *in vitro* toxicity testing with the agent if:

    i) the delta percent priming in the cancerous cells is greater than 20 percent, greater than 30 percent, greater than 40 percent, greater than 50 percent, greater than 60 percent, greater than 70 percent, greater than 80 percent, greater than 90 percent, greater than 95 percent, or 100 percent; or
    ii) the delta percent priming in the cancerous cells is greater than the delta percent priming in the non-cancerous cells; or
    iii) the delta percent priming in the cancerous cells is at least 10%, at least 20%, at least 30%, at least 40%, at least 50%, at least 60%, at least 70%, at least 80%, at least 90%, at least 100%, at least 150%, or at least 200%, greater than the delta percent priming in the non-cancerous cells; or
    iv) the delta percent priming in the cancerous cells is at least 2-fold, at least 3-fold, at least 4-fold, at least 5-fold, at least 6-fold, at least 7-fold, at least 8-fold, at least 9-fold, or at least 10-fold greater than the delta percent priming in the non-cancerous cells.

**5.** The method of any one of claims 3-4, wherein the cancerous cells are primary human tumor cells; or wherein the cancerous cells are obtained from a patient derived xenograft (PDX).

**6.** The method of any one of claims 1-5, wherein:

i) the plurality of agents includes 20 or more agents; and/or
ii) the plurality of agents includes 100 or more agents; and/or
iii) each test aliquot is contacted with its respective agent for 24 hours or less; and/or
iv) the permeabilized cells are contacted with the pro-apoptotic BH3 domain peptide for 3 hours or less; and/or
v) the cells in each of the test aliquots are permeabilized with digitonin or saponin.

**7.** The method of any one of claims 1-6, wherein the percent BH3 domain peptide-induced MOMP is determined by one or more of:

i) contacting the cells with a potentiometric dye and measuring the emission of said potentiometric dye, wherein the potentiometric dye is selected from the group consisting of 5,5',6,6'-tetrachloro-1,1',3,3'-tetraethylbenzimidazolylcarbocyanine iodide (JC-1), dihydrorhodamine 123, tetramethylrhodamine methyl ester (TMRM), or tetramethylrhodamine ethyl ester (TMRE);
ii) measuring the release of a molecule from the mitochondrial intermembrane space, wherein the molecule from the mitochondrial intermembrane space is selected from the group consisting of cytochrome c, SMAC/Diablo, Omi, adenylate kinase-2, or apoptosis-inducing factor; and
iii) measuring the retention of a molecule from the mitochondrial intermembrane space, wherein the molecule from the mitochondrial intermembrane space is selected from the group consisting of cytochrome c, second mitochondria-derived activator of caspases (SMAC)/ Diablo, Omi, adenylate kinase-2, or apoptosis-

inducing factor.

**8.** The method of any one of claims 1-7, wherein:

i) the BH3 domain peptide is derived from the BH3 domain of a BH3 interacting domain death agonist (BID), a Bcl-2 interacting mediator of cell death (BIM), a Bcl-2-associated death promoter (BAD), a Noxa, a p53 up-regulated modulator of apoptosis (PUMA), a Bcl-2-modifying factor (BMF), or a harakiri (HRK) polypeptide; and/or
ii) the BH3 domain peptide is selected from the group consisting of SEQ ID NO: 1-16; and/or
iii) an agent from the plurality of agents comprises one or more compounds; and/or
iv) an agent from the plurality of agents is an anticancer agent; and/or
v) an agent from the plurality of agents is a chemotherapeutic agent; and/or
vi) one or more of the plurality of agents are selected from small organic molecules, small inorganic molecules, peptides, proteins, protein analogs, enzymes, nucleic acids, nucleic acid analogs, antibodies, antigens, hormones, lipids, polysaccharides, growth factors, viruses, cells, bioactive agents, pharmaceutical agents, and combinations and prodrugs thereof; and/or
vii)one or more of the plurality of agents are selected from gases, fine particles, radiation, electromagnetic radiation, and aerosols.

**9.** A method of predicting toxicity of an agent to a tissue, said method comprising:

a) providing an agent;
b) providing a test aliquot of each of a plurality of cell samples, wherein each cell sample comprises non-cancerous cells previously obtained from a tissue of a subject:
c) separately contacting each test aliquot with the agent;
d) providing a control aliquot of each of the plurality of cell samples, wherein the control aliquot is not contacted with the agent;
e) permeabilizing the cells in each test aliquot and control aliquot;
f) contacting the permeabilized cells with a pro-apoptotic BH3 domain peptide;

g) determining a value for percent BH3 domain peptide-induced MOMP in the cells in each test aliquot;

h) determining a value for percent BH3 domain peptide-induced MOMP in the cells in each control aliquot; and

i) determining a value for delta percent priming for each test aliquot, wherein delta percent priming is the difference between the value for percent MOMP determined in (g) and the value for percent MOMP determined in (h),

wherein a delta percent priming of greater than 20 percent indicates that the agent is toxic to the tissue from which the cells were obtained.

10. The method of claim 9, further comprising conducting *in vivo* toxicity testing with the agent in a non-human animal or *in vitro* toxicity testing with the agent if the delta percent priming is 20 percent or less, 18 percent or less, 16 percent or less, 14 percent or less, 12 percent or less, 10 percent or less, 8 percent or less, 6 percent or less, 4 percent or less, 2 percent or less, or 1 percent or less.

11. The method of claim 9 or 10, wherein:

i) the non-cancerous cells are healthy cells; and/or

ii) the cells are mammalian cells; and/or

iii) the cells are derived from samples obtained by biopsy or autopsy of an animal; and/or

iv) the cells in each test aliquot are contacted with the agent for 24 hours or less; and/or

v) the permeabilized cells are contacted with the pro-apoptotic BH3 domain peptide for 3 hours or less; and/or

vi) the cells are permeabilized with digitonin or saponin.

12. The method of any one of claims 9-11, wherein the percent BH3 domain peptide-induced MOMP is determined by one or more of:

i) contacting the cells with a potentiometric dye and measuring the emission of said potentiometric dye, wherein the potentiometric dye is selected from the group consisting of 5,5',6,6'-tetrachloro-1,1',3,3'-tetraethylbenzimidazolylcarbocyanine iodide (JC-1), dihydrorhodamine 123, tetramethylrhodamine methyl ester (TMRM), or tetramethylrhodamine ethyl ester (TMRE);

ii) measuring the release of a molecule from the mitochondrial intermembrane space, wherein the molecule from the mitochondrial intermembrane space is selected from the group consisting of cytochrome c, SMAC/Diablo, Omi, adenylate kinase-2, or apoptosis-inducing factor; and

iii) measuring the retention of a molecule from the mitochondrial intermembrane space, wherein the molecule from the mitochondrial intermembrane space is selected from the group consisting of cytochrome c, SMAC/Diablo, Omi, adenylate kinase-2, or apoptosis-inducing factor.

13. The method of any one of claims 9-12, wherein:

i) the BH3 domain peptide is derived from the BH3 domain of a BID, a BIM, a BAD, a NOXA, a PUMA, a BMF, or an HRK polypeptide; and/or

ii) the BH3 domain peptide is selected from the group consisting of SEQ ID NO: 1-16; and/or

iii) the agent comprises one or more compounds; and/or

iv) the agent is an anticancer agent; and/or

v) the agent is a chemotherapeutic agent; and/or

vi) the agent is a small organic molecule, small inorganic molecule, peptide, protein, protein analog, enzyme, nucleic acid, nucleic acid analog, antibody, antigen, hormone, lipid, polysaccharide, growth factor, virus, cell, bioactive agent, pharmaceutical agent, or some combination or prodrug thereof; and/or

vii) the agent is gas, fine particles, radiation, electromagnetic radiation, or aerosol.

14. A method of predicting toxicity of an agent, said method comprising:

a) providing one or more cell samples, wherein each cell sample comprises non-cancerous cells previously obtained from a tissue from a non-human animal that had been contacted with a sublethal dose of an agent and then sacrificed;

b) providing a test aliquot of each of the one or more cell samples;

c) separately contacting each test aliquot with the agent;

d) providing a control aliquot of each of the one or more cell samples, wherein the control aliquot is not contacted with the agent;

e) permeabilizing the cells in each aliquot;

f) contacting the permeabilized cells with a pro-apoptotic BH3 domain peptide;

g) determining a value for percent BH3 domain peptide-induced MOMP in the cells in the test aliquot;

h) determining a value for percent BH3 domain peptide-induced MOMP in the cells in the control aliquot; and

i) determining a value for delta percent priming for the test aliquot, wherein delta percent priming is the difference between the value for percent MOMP determined in (g) and the value for percent MOMP determined in (h),

wherein a delta percent priming of greater than 20 percent indicates that the agent is toxic to the tissue of the non-human animal from which the cells were obtained.

**15.** The method of claim 14, wherein:

i) the non-human animal that has been contacted with a sublethal dose of an agent is a mammal, optionally wherein the mammal is a non-human primate or a rodent; and/or

ii) the non-human animal has been contacted with the agent by injection, inhalation, topical administration, or oral administration; and/or

iii) the non-human animal is contacted with the agent for 24 hours or less; and/or

iv) the permeabilized cells are contacted with the pro-apoptotic BH3 domain peptide for 3 hours or less; and/or

v) the cells are permeabilized with digitonin or saponin; and/or

vi) the BH3 domain peptide is derived from the BH3 domain of a BID, a BIM, a BAD, a NOXA, a PUMA, a BMF, or an HRK polypeptide; and/or

vii) the BH3 domain peptide is selected from the group consisting of SEQ ID NO: 1-16; and/or

viii) the agent comprises one or more compounds; and/or

ix) the agent is an anticancer agent; and/or

x) the agent is a chemotherapeutic agent; and/or

xi) the agent is a small organic molecule, small inorganic molecule, peptide, protein, protein analog, enzyme, nucleic acid, nucleic acid analog, antibody, antigen, hormone, lipid, polysaccharide, growth factor, virus, cell, bioactive agent, pharmaceutical agent, or some combination or prodrug thereof; and/or

xii) the agent is gas, fine particles, radiation, electromagnetic radiation, or aerosol.

**Patentansprüche**

**1.** Verfahren zum Screening einer Vielzahl von Mitteln zum Bestimmen von Toxizität, das Verfahren umfassend:

a) Bereitstellen einer Vielzahl von Mitteln;

b) Bereitstellen eines Test-Aliquots einer Probe von Zellen für jedes der Vielzahl von Mitteln, wobei die Probe von Zellen nicht-krebsartige Zellen sind, die zuvor von einem Subjekt erlangt wurden;

c) separates Inkontaktbringen von jedem Test-Aliquot mit seinem jeweiligen Mittel;

d) Bereitstellen eines Kontroll-Aliquots der Probe von Zellen, wobei das Kontroll-Aliquot nicht mit einem beliebigen der Vielzahl von Mitteln in Kontakt gebracht wird;

e) Permeabilisieren der Zellen in jedem Aliquot;

f) Inkontaktbringen der permeabilisierten Zellen mit einem pro-apoptotischen Peptid der Domäne Bcl-2-Homologie 3 (BH3);

g) Bestimmen eines Werts für eine durch das Peptid der Domäne BH3 induzierte prozentuale Permeabilisierung der äußeren mitochondrialen Membran (MOMP) in den Zellen in jedem der Test-Aliquots;

h) Bestimmen eines Werts für eine durch das Peptid der Domäne BH3 induzierte prozentuale MOMP in den Zellen in dem Kontroll-Aliquot;

i) Bestimmen eines Werts für Delta-Prozent-Priming für jedes der Test-Aliquots, wobei Delta-Prozent-Priming die Differenz zwischen dem in (g) bestimmten Wert für prozentuale MOMP und dem in (h) bestimmten Wert für prozentuale MOMP ist,

wobei ein Delta-Prozent-Priming von mehr als 20 Prozent angibt, dass das Mittel, mit dem die Zellen im Test-Aliquot in Kontakt gebracht wurden, für die Zellen toxisch ist.

**2.** Verfahren nach Anspruch 1, ferner umfassend ein Durchführen von *in* vivo-Toxizitätstests mit dem Mittel bei einem nicht-menschlichen Tier oder von *in* vitro-Toxizitätstests mit dem Mittel, wenn das Delta-Prozent-Priming 20 Prozent oder weniger, 18 Prozent oder weniger, 16 Prozent oder weniger, 14 Prozent oder weniger, 12 Prozent oder weniger, 10 Prozent oder weniger, 8 Prozent oder weniger, 6 Prozent oder weniger, 4 Prozent oder weniger, 2 Prozent oder weniger oder 1 Prozent oder weniger ist; und/oder wobei die nicht-krebsartigen Zellen gesunde Zellen sind.

3. Verfahren nach einem der Ansprüche 1-2, ferner umfassend:

j) Bereitstellen eines Test-Aliquots einer Probe von krebsartigen Zellen, die zuvor von einem Subjekt erlangt wurde, für jedes der Vielzahl von Mitteln;

k) separates Inkontaktbringen von jedem Test-Aliquot mit seinem jeweiligen Mittel;

l) Bereitstellen eines Kontroll-Aliquots der Probe von krebsartigen Zellen, wobei das Kontroll-Aliquot nicht mit einem beliebigen der Vielzahl von Mitteln in Kontakt gebracht wird;

m) Permeabilisieren der krebsartigen Zellen in jedem Aliquot;

n) Inkontaktbringen der permeabilisierten krebsartigen Zellen mit einem pro-apoptotischen Peptid der Domäne BH3;

o) Bestimmen eines Werts für eine durch das Peptid der Domäne BH3 induzierte prozentuale MOMP in den krebsartigen Zellen in jedem der Test-Aliquots;

p) Bestimmen eines Werts für eine durch das Peptid der Domäne BH3 induzierte prozentuale MOMP in den krebsartigen Zellen in dem Kontroll-Aliquot; und

q) Bestimmen eines Werts für Delta-Prozent-Priming für jedes der Test-Aliquots, wobei Delta-Prozent-Priming die Differenz zwischen dem in (o) bestimmten Wert für prozentuale MOMP und dem in (p) bestimmten Wert für prozentuale MOMP ist,

wobei ein Delta-Prozent-Priming von mehr als 20 Prozent angibt, dass das Mittel, mit dem die krebsartigen Zellen im Test-Aliquot in Kontakt gebracht wurden, für die krebsartigen Zellen toxisch ist.

4. Verfahren nach Anspruch 3, ferner umfassend ein Durchführen von *in-vivo*-Toxizitätstests mit dem Mittel bei einem nicht-menschlichen Tier oder von *in-vitro*-Toxizitätstests mit dem Mittel, wenn:

i) das Delta-Prozent-Priming in den krebsartigen Zellen größer als 20 Prozent, größer als 30 Prozent, größer als 40 Prozent, größer als 50 Prozent, größer als 60 Prozent, größer als 70 Prozent, größer als 80 Prozent, größer als 90 Prozent, größer als 95 Prozent oder 100 Prozent ist; oder

ii) das Delta-Prozent-Priming in den krebsartigen Zellen größer als das Delta-Prozent-Priming in den nicht-krebsartigen Zellen ist; oder

iii) das Delta-Prozent-Priming in den krebsartigen Zellen mindestens 10 %, mindestens 20 %, mindestens 30 %, mindestens 40 %, mindestens 50 %, mindestens 60 %, mindestens 70 %, mindestens 80 %, mindestens 90 %, mindestens 100 %, mindestens 150 % oder mindestens 200 % größer ist als das Delta-Prozent-Priming in den nicht-krebsartigen Zellen ist; oder

iv) das Delta-Prozent-Priming in den krebsarigen Zellen mindestens 2-fach, mindestens 3-fach, mindestens 4-fach, mindestens 5-fach, mindestens 6-fach, mindestens 7-fach, mindestens 8-fach, mindestens 9-fach oder mindestens 10-fach größer ist als das Delta-Prozent-Priming in den nicht-krebsartigen Zellen.

5. Verfahren nach einem der Ansprüche 3-4, wobei die krebsartigen Zellen primäre menschliche Tumorzellen sind; oder wobei die krebsartigen Zellen aus einem patientenabgeleiteten Xenograft (PDX) erlangt werden.

6. Verfahren nach einem der Ansprüche 1-5, wobei:

i) die Vielzahl der Mittel 20 oder mehr Mittel beinhaltet; und/oder

ii) die Vielzahl der Mittel 100 oder mehr Mittel beinhaltet; und/oder

iii) jedes Test-Aliquot 24 Stunden oder weniger mit dem jeweiligen Mittel in Kontakt gebracht wird; und/oder

iv) die permeabilisierten Zellen 3 Stunden oder weniger mit dem pro-apoptotischen Peptid der Domäne BH3 in Kontakt gebracht werden; und/oder

v) die Zellen in jedem der Test-Aliquots mit Digitonin oder Saponin permeabilisiert werden.

7. Verfahren nach einem der Ansprüche 1-6, wobei die durch das Peptid der Domäne BH3 induzierte prozentuale MOMP durch eines oder mehrere von Folgenden bestimmt wird:

i) Inkontaktbringen der Zellen mit einem potentiometrischen Farbstoff und Messen der Emission des potentiometrischen Farbstoffs, wobei der potentiometrische Farbstoff ausgewählt ist aus der Gruppe bestehend aus 5,5',6,6'-Tetrachlor-1,1',3,3'-tetraethylbenzimidazolylcarbocyaninjodid (JC-1), Dihydrorhodamin 123, Tetramethylrhodaminmethylester (TMRM) oder Tetramethylrhodaminethylester (TMRE);

ii) Messen der Freisetzung eines Moleküls aus dem mitochondrialen Intermembranraum, wobei das Molekül aus dem mitochondrialen Intermembranraum ausgewählt ist aus der Gruppe bestehend aus Cytochrom c, SMAC/Diablo, Omi, Adenylatkinase-2 oder Apoptoseinduzierendem Faktor; und

iii) Messen der Retention eines Moleküls aus dem mitochondrialen Intermembranraum, wobei das Molekül aus dem mitochondrialen Intermembranraum ausgewählt ist aus der Gruppe bestehend aus Cytochrom c, dem zweiten Mitochondrien-abgeleiteten Aktivator von Caspasen (SMAC)/Diablo, Omi, Adenylatkinase-2 oder dem Apoptose-induzierenden Faktor.

8. Verfahren nach einem der Ansprüche 1-7, wobei:

i) das Peptid der Domäne BH3 abgeleitet ist von der Domäne BH3 eines Polypeptids eines BH3-Interaktionsdomänen-Todesagonisten (BID), eines Bcl-2-interagierenden Zelltodvermittlers (BIM), eines Bcl-2-assoziierten Todespromotors (BAD), einer Noxa, eines p53-aufwärtsregulierten Apoptosemodulators (PUMA), eines Bcl-2-modifizierenden Faktors (BMF) oder eines Harakiri (HRK); und/oder

ii) das Peptid der Domäne BH3 ausgewählt ist aus der Gruppe bestehend aus SEQ ID NO: 1-16; und/oder

iii) ein Mittel aus der Vielzahl der Mittel eine oder mehrere Verbindungen umfasst; und/oder

iv) ein Mittel aus der Vielzahl von Mitteln ein Mittel gegen Krebs ist; und/oder

v) ein Mittel aus der Vielzahl der Mittel ein Chemotherapeutikum ist; und/oder

vi) eines oder mehrere der Vielzahl von Mitteln ausgewählt sind aus kleinen organischen Molekülen, kleinen anorganischen Molekülen, Peptiden, Proteinen, Proteinanaloga, Enzymen, Nukleinsäuren, Nukleinsäureanaloga, Antikörpern, Antigenen, Hormonen, Lipiden, Polysacchariden, Wachstumsfaktoren, Viren, Zellen, bioaktiven Mitteln, pharmazeutischen Mitteln und Kombinationen und Prodrugs davon; und/oder

vii) eines oder mehrere der Vielzahl von Mitteln ausgewählt sind aus Gasen, feinen Partikeln, Strahlung, elektromagnetischer Strahlung und Aerosolen.

9. Verfahren zum Vorhersagen von Toxizität eines Mittels für ein Gewebe, das Verfahren umfassend:

a) Bereitstellen eines Mittels;

b) Bereitstellen eines Test-Aliquots von jeder einer Vielzahl von Proben von Zellen, wobei jede Probe von Zellen nicht-krebsartige Zellen umfasst, die zuvor aus einem Gewebe eines Subjekts erlangt wurden;

c) separates Inkontaktbringen von jedem Test-Aliquot mit dem Mittel;

d) Bereitstellen eines Kontroll-Aliquots von jeder der Vielzahl von Proben von Zellen, wobei das Kontroll-Aliquot nicht mit dem Mittel in Kontakt gebracht wird;

e) Permeabilisieren der Zellen in jedem Test-Aliquot und Kontroll-Aliquot;

f) Inkontaktbringen der permeabilisierten Zellen mit einem pro-apoptotischen Peptid der Domäne BH3;

g) Bestimmen eines Werts für eine durch das Peptid der Domäne BH3 induzierte prozentuale MOMP in den Zellen in jedem Test-Aliquot;

h) Bestimmen eines Werts für eine durch das Peptid der Domäne BH3 induzierte prozentuale MOMP in den Zellen in jedem Kontroll-Aliquot;

i) Bestimmen eines Werts für Delta-Prozent-Priming für jedes Test-Aliquot, wobei Delta-Prozent-Priming die Differenz zwischen dem in (g) bestimmten Wert für prozentuale MOMP und dem in (h) bestimmten Wert für prozentuale MOMP ist,

wobei ein Delta-Prozent-Priming von mehr als 20 Prozent angibt, dass das Mittel für das Gewebe, aus dem die Zellen erlangt wurden, toxisch ist.

10. Verfahren nach Anspruch 9, ferner umfassend ein Durchführen von *in vivo*-Toxizitätstests mit dem Mittel bei einem nicht-menschlichen Tier oder von *in vitro*-Toxizitätstests mit dem Mittel, wenn das Delta-Prozent-Priming 20 Prozent oder weniger, 18 Prozent oder weniger, 16 Prozent oder weniger, 14 Prozent oder weniger, 12 Prozent oder weniger, 10 Prozent oder weniger, 8 Prozent oder weniger, 6 Prozent oder weniger, 4 Prozent oder weniger, 2 Prozent oder weniger oder 1 Prozent oder weniger ist.

11. Verfahren nach Anspruch 9 oder 10, wobei:

i) die nicht-krebsartigen Zellen gesunde Zellen sind; und/oder

ii) die Zellen Säugetierzellen sind; und/oder

iii) die Zellen aus Proben abgeleitet sind, die durch Biopsie oder Autopsie eines Tiers erlangt wurden; und/oder

iv) die Zellen in jedem Test-Aliquot 24 Stunden oder weniger mit dem Mittel in Kontakt gebracht werden; und/oder

v) die permeabilisierten Zellen 3 Stunden oder weniger mit dem pro-apoptotischen Peptid der Domäne BH3 in Kontakt gebracht werden; und/oder

vi) die Zellen mit Digitonin oder Saponin permeabilisiert werden.

**12.** Verfahren nach einem der Ansprüche 9-11, wobei die durch das Peptid der Domäne BH3 induzierte prozentuale MOMP durch eines oder mehrere von Folgenden bestimmt wird:

i) Inkontaktbringen der Zellen mit einem potentiometrischen Farbstoff und Messen der Emission des potentiometrischen Farbstoffs, wobei der potentiometrische Farbstoff ausgewählt ist aus der Gruppe bestehend aus 5,5',6,6'-Tetrachlor-1,1',3,3'-tetraethylbenzimidazolylcarbocyaninjodid (JC-1), Dihydrorhodamin 123, Tetramethylrhodaminmethylester (TMRM) oder Tetramethylrhodaminethylester (TMRE);

ii) Messen der Freisetzung eines Moleküls aus dem mitochondrialen Intermembranraum, wobei das Molekül aus dem mitochondrialen Intermembranraum ausgewählt ist aus der Gruppe bestehend aus Cytochrom c, SMAC/Diablo, Omi, Adenylatkinase-2 oder Apoptoseinduzierendem Faktor; und

iii) Messen der Retention eines Moleküls aus dem mitochondrialen Intermembranraum, wobei das Molekül aus dem mitochondrialen Intermembranraum ausgewählt ist aus der Gruppe bestehend aus Cytochrom c, SMAC/Diablo, Omi, Adenylatkinase-2 oder Apoptoseinduzierendem Faktor.

**13.** Verfahren nach einem der Ansprüche 9-12, wobei:

i) das Peptid der Domäne BH3 abgeleitet ist von der Domäne BH3 eines BID-, eines BIM-, eines BAD-, eines NOXA-, eines PUMA-, eines BMF- oder eines HRK-Polypeptids; und/oder

ii) das Peptid der Domäne BH3 ausgewählt ist aus der Gruppe bestehend aus SEQ ID NO: 1-16; und/oder

iii) das Mittel eine oder mehrere Verbindungen umfasst; und/oder

iv) das Mittel ein Mittel gegen Krebs ist; und/oder

v) das Mittel ein Chemotherapeutikum ist; und/oder

vi) das Mittel ein kleines organisches Molekül, ein kleines anorganisches Molekül, ein Peptid, ein Protein, ein Proteinanalogon, ein Enzym, eine Nukleinsäure, ein Nukleinsäureanalogon, ein Antikörper, ein Antigen, ein Hormon, ein Lipid, ein Polysaccharid, ein Wachstumsfaktor, ein Virus, eine Zelle, ein bioaktives Mittel, ein pharmazeutisches Mittel oder eine Kombination oder ein Prodrug davon ist; und/oder

vii) das Mittel Gas, feine Partikel, Strahlung, elektromagnetische Strahlung oder ein Aerosol ist.

**14.** Verfahren zum Vorhersagen von Toxizität eines Mittels, das Verfahren umfassend:

a) Bereitstellen einer oder mehrerer Proben von Zellen, wobei jede Probe von Zellen nicht-krebsartige Zellen umfasst, die zuvor aus einem Gewebe eines nicht-menschlichen Tiers erlangt wurden, das mit einer subletalen Dosis eines Mittels in Kontakt gebracht und dann geopfert wurde;

b) Bereitstellen eines Test-Aliquots von jeder der einen oder der mehreren Proben von Zellen;

c) separates Inkontaktbringen von jedem Test-Aliquot mit dem Mittel;

d) Bereitstellen eines Kontroll-Aliquots von jeder der einen oder der mehreren Proben von Zellen, wobei das Kontroll-Aliquot nicht mit dem Mittel in Kontakt gebracht wird;

e) Permeabilisieren der Zellen in jedem Aliquot;

f) Inkontaktbringen der permeabilisierten Zellen mit einem pro-apoptotischen Peptid der Domäne BH3;

g) Bestimmen eines Werts für eine durch das Peptid der Domäne BH3 induzierte prozentuale MOMP in den Zellen in dem Test-Aliquot;

h) Bestimmen eines Werts für eine durch das Peptid der Domäne BH3 induzierte prozentuale MOMP in den Zellen in dem Kontroll-Aliquot;

i) Bestimmen eines Werts für Delta-Prozent-Priming für das Test-Aliquot, wobei Delta-Prozent-Priming die Differenz zwischen dem in (g) bestimmten Wert für prozentuale MOMP und dem in (h) bestimmten Wert für prozentuale MOMP ist,

wobei ein Delta-Prozent-Priming von mehr als 20 Prozent angibt, dass das Mittel für das Gewebe des nicht-menschlichen Tiers, aus dem die Zellen erlangt wurden, toxisch ist.

**15.** Verfahren nach Anspruch 14, wobei:

i) das nichtmenschliche Tier, das mit einer subletalen Dosis eines Mittels in Kontakt gebracht wird, ein Säugetier ist, optional wobei das Säugetier ein nicht-menschlicher Primat oder ein Nagetier ist; und/oder

ii) das nichtmenschliche Tier durch Injektion, Inhalation, topische Verabreichung oder orale Verabreichung mit dem Mittel in Kontakt gebracht wird; und/oder

iii) das nicht-menschliche Tier 24 Stunden oder weniger mit dem Mittel in Kontakt gebracht wird; und/oder

iv) die permeabilisierten Zellen 3 Stunden oder weniger mit dem pro-apoptotischen Peptid der Domäne BH3 in Kontakt gebracht werden; und/oder

v) die Zellen mit Digitonin oder Saponin permeabilisiert werden; und/oder

vi) das Peptid der Domäne BH3 abgeleitet ist von der Domäne BH3 eines BID-, eines BIM-, eines BAD-, eines NOXA-, eines PUMA-, eines BMF- oder eines HRK-Polypeptids; und/oder

vii) das Peptid der Domäne BH3 ausgewählt ist aus der Gruppe bestehend aus SEQ ID NO: 1-16; und/oder

viii) das Mittel eine oder mehrere Verbindungen umfasst; und/oder

ix) das Mittel ein Mittel gegen Krebs ist; und/oder

x) das Mittel ein Chemotherapeutikum ist; und/oder

xi) das Mittel ein kleines organisches Molekül, ein kleines anorganisches Molekül, ein Peptid, ein Protein, ein Proteinanalogon, ein Enzym, eine Nukleinsäure, ein Nukleinsäureanalogon, ein Antikörper, ein Antigen, ein Hormon, ein Lipid, ein Polysaccharid, ein Wachstumsfaktor, ein Virus, eine Zelle, ein bioaktives Mittel, ein pharmazeutisches Mittel oder eine Kombination oder ein Prodrug davon ist; und/oder

xii) das Mittel Gas, feine Partikel, Strahlung, elektromagnetische Strahlung oder ein Aerosol ist.

## Revendications

1. Méthode de criblage d'une pluralité d'agents permettant de déterminer la toxicité, ladite méthode comprenant :

a) la fourniture d'une pluralité d'agents ;

b) la fourniture d'une aliquote d'essai d'un échantillon de cellules pour chacun de la pluralité d'agents, ledit échantillon de cellules étant constitué de cellules non cancéreuses préalablement prélevées chez un sujet ;

c) la mise en contact séparément de chaque aliquote d'essai avec son agent respectif ;

d) la fourniture d'une aliquote témoin de l'échantillon de cellules, ladite aliquote témoin n'étant pas mise en contact avec l'un quelconque de la pluralité d'agents ;

e) la perméabilisation des cellules dans chaque aliquote ;

f) la mise en contact des cellules perméabilisées avec un peptide pro-apoptotique du domaine d'homologie 3 de Bcl-2 (BH3) ;

g) la détermination d'une valeur du pourcentage de la perméabilisation de la membrane externe mitochondriale (MOMP) induite par le peptide du domaine BH3 dans les cellules dans chacune des aliquotes d'essai ;

h) la détermination d'une valeur du pourcentage de la MOMP induite par le peptide du domaine BH3 dans les cellules dans l'aliquote témoin ; et

i) la détermination d'une valeur du pourcentage d'amorçage delta pour chacune des aliquotes d'essai, ledit pourcentage d'amorçage delta étant la différence entre la valeur du pourcentage de la MOMP déterminée en (g) et la valeur du pourcentage de la MOMP déterminée en (h),

un pourcentage d'amorçage delta supérieur à 20 pour cent indiquant que l'agent avec lequel les cellules dans l'aliquote d'essai ont été mises en contact est toxique pour les cellules.

2. Méthode selon la revendication 1, comprenant en outre la réalisation d'essais de toxicité *in vivo* avec l'agent chez un animal non humain ou d'essais de toxicité *in vitro* avec l'agent si le pourcentage d'amorçage delta est inférieur ou égal à 20 pour cent, inférieur ou égal à 18 pour cent, inférieur ou égal à 16 pour cent, inférieur ou égal à 14 pour cent, inférieur ou égal à 12 pour cent, inférieur ou égal à 10 pour cent, inférieur ou égal à 8 pour cent, inférieur ou égal à 6 pour cent, inférieur ou égal à 4 pour cent, inférieur ou égal à 2 pour cent, ou inférieur ou égal à 1 pour cent ; et/ou lesdites cellules non cancéreuses étant des cellules saines.

3. Méthode selon l'une quelconque des revendications 1 à 2, comprenant en outre :

j) la fourniture d'une aliquote d'essai d'un échantillon de cellules cancéreuses préalablement prélevé chez un sujet pour chacun de la pluralité d'agents ;

k) la mise en contact séparément de chaque aliquote d'essai avec son agent respectif ;

1) la fourniture d'une aliquote témoin de l'échantillon de cellules cancéreuses, ladite aliquote témoin n'étant pas mise en contact avec l'un quelconque de la pluralité d'agents ;

m) la perméabilisation des cellules cancéreuses dans chaque aliquote ;

n) la mise en contact des cellules cancéreuses perméabilisées avec un peptide du domaine BH3 pro-apoptotique ;

o) la détermination d'une valeur du pourcentage de la MOMP induite par le peptide du domaine BH3 dans les cellules cancéreuses dans chacune des aliquotes d'essai ;

p) la détermination d'une valeur du pourcentage de la MOMP induite par le peptide du domaine BH3 dans les cellules cancéreuses dans l'aliquote témoin ; et

q) la détermination d'une valeur du pourcentage d'amorçage delta pour chacune des aliquotes d'essai, ledit pourcentage d'amorçage delta étant la différence entre la valeur du pourcentage de la MOMP déterminée en (o) et la valeur du pourcentage de la MOMP déterminée en (p),

un pourcentage d'amorçage delta supérieur à 20 pour cent indiquant que l'agent avec lequel les cellules cancéreuses dans l'aliquote d'essai ont été mises en contact est toxique pour les cellules cancéreuses.

4. Méthode selon la revendication 3, comprenant en outre la réalisation d'essais de toxicité *in vivo* avec l'agent chez un animal non humain ou dans des essais de toxicité *in vitro* avec l'agent si :

i) le pourcentage d'amorçage delta dans les cellules cancéreuses est supérieur à 20 pour cent, supérieur à 30 pour cent, supérieur à 40 pour cent, supérieur à 50 pour cent, supérieur à 60 pour cent, supérieur à 70 pour cent, supérieur à 80 pour cent, supérieur à 90 pour cent, supérieur à 95 pour cent, ou 100 pour cent ; ou

ii) le pourcentage d'amorçage delta dans les cellules cancéreuses est supérieur au pourcentage d'amorçage delta dans les cellules non cancéreuses ; ou

iii) le pourcentage d'amorçage delta dans les cellules cancéreuses est supérieur ou égal à 10 %, supérieur ou égal à 20 %, supérieur ou égal à 30 %, supérieur ou égal à 40 %, supérieur ou égal à 50 %, supérieur ou égal à 60 %, supérieur ou égal à 70 %, supérieur ou égal à 80 %, supérieur ou égal à 90 %, supérieur ou égal à 100 %, supérieur ou égal à 150 %, ou supérieur ou égal à 200 %, supérieur au pourcentage d'amorçage delta dans les cellules non cancéreuses ; ou

iv) le pourcentage d'amorçage delta dans les cellules cancéreuses est supérieur ou égal à 2 fois, supérieur ou égal à 3 fois, supérieur ou égal à 4 fois, supérieur ou égal à 5 fois, supérieur ou égal à 6 fois, supérieur ou égal à 7 fois, supérieur ou égal à 8 fois, supérieur ou égal à 9 fois ou supérieur ou égal à 10 fois le pourcentage d'amorçage delta dans les cellules non cancéreuses.

5. Méthode selon l'une quelconque des revendications 3 à 4, lesdites cellules cancéreuses étant des cellules tumorales humaines primaires ; ou lesdites cellules cancéreuses étant prélevées sur une xénogreffe dérivée d'un patient (PDX).

6. Méthode selon l'une quelconque des revendications 1 à 5 :

i) ladite pluralité d'agents comprenant 20 agents ou plus ; et/ou

ii) ladite pluralité d'agents comprenant 100 agents ou plus ; et/ou

iii) chaque aliquote d'essai étant mise en contact avec son agent respectif pendant 24 heures ou moins ; et/ou

iv) lesdites cellules perméabilisées étant mises en contact avec le peptide du domaine BH3 pro-apoptotique pendant 3 heures ou moins ; et/ou

v) lesdites cellules de chacune des aliquotes d'essai étant perméabilisées avec de la digitonine ou de la saponine.

7. Méthode selon l'une quelconque des revendications 1 à 6, ledit pourcentage de la MOMP induite par le peptide du domaine BH3 étant déterminé par une ou plusieurs parmi :

i) la mise en contact des cellules avec un colorant potentiométrique et la mesure de l'émission dudit colorant potentiométrique, ledit colorant potentiométrique étant choisi dans le groupe constitué par l'iodure de 5,5',6,6'-tétrachloro-1,1',3,3'-tétraéthylbenzimidazolylcarbocyanine (JC-1), la dihydrorhodamine 123, l'ester méthylique de tétraméthylrhodamine (TMRM) ou l'ester éthylique de tétraméthylrhodamine (TMRE) ;

ii) la mesure de la libération d'une molécule provenant de l'espace intermembranaire mitochondrial, ladite molécule provenant de l'espace intermembranaire mitochondrial étant choisie dans le groupe constitué par le cytochrome c, le SMAC/Diablo, l'Omi, l'adénylate kinase-2 ou le facteur inducteur de l'apoptose ; et

iii) la mesure de la rétention d'une molécule provenant de l'espace intermembranaire mitochondrial, ladite molécule provenant de l'espace intermembranaire mitochondrial étant choisie dans le groupe constitué par le cytochrome c, le deuxième activateur de caspases dérivé de mitochondries (SMAC)/Diablo, l'Omi, l'adénylate kinase-2, ou le facteur inducteur de l'apoptose.

8. Méthode selon l'une quelconque des revendications 1 à 7 :

i) ledit peptide du domaine BH3 étant dérivé du domaine BH3 d'un agoniste de mort à domaine d'interaction BH3 (BID), d'un médiateur de mort cellulaire interagissant avec Bcl-2 (BIM), d'un promoteur de mort associé à Bcl-2 (BAD), d'une Noxa, d'un modulateur de l'apoptose régulé positivement par p53 (PUMA), d'un facteur modificateur de Bcl-2 (BMF) ou d'un polypeptide harakiri (HRK) ; et/ou

ii) ledit peptide du domaine BH3 étant choisi dans le groupe constitué par les SEQ ID n° : 1 à 16 ; et/ou

iii) un agent parmi ladite pluralité d'agents comprenant un ou plusieurs composés ; et/ou

iv) un agent parmi ladite pluralité d'agents étant un agent anticancéreux ; et/ou

v) un agent parmi ladite pluralité d'agents étant un agent chimiothérapeutique ; et/ou

vi) un ou plusieurs agents parmi ladite pluralité d'agents étant choisis parmi les petites molécules organiques, les petites molécules inorganiques, les peptides, les protéines, les analogues de protéines, les enzymes, les acides nucléiques, les analogues d'acide nucléique, les anticorps, les antigènes, les hormones, les lipides, les polysaccharides, les facteurs de croissance, les virus, les cellules, les agents bioactifs, les agents pharmaceutiques, et les combinaisons et promédicaments de ceux-ci ; et/ou

vii) un ou plusieurs agents parmi ladite pluralité d'agents étant choisis parmi les gaz, les particules fines, un rayonnement, un rayonnement électromagnétique et les aérosols.

9. Méthode de prédiction de la toxicité d'un agent pour un tissu, ladite méthode comprenant :

a) la fourniture d'un agent ;

b) la fourniture d'une aliquote d'essai de chacun d'une pluralité d'échantillons de cellules, chaque échantillon de cellules comprenant des cellules non cancéreuses préalablement prélevées sur un tissu d'un sujet ;

c) la mise en contact séparément de chaque aliquote d'essai avec l'agent ;

d) la fourniture d'une aliquote témoin de chacun de la pluralité d'échantillons de cellules, ladite aliquote témoin n'étant pas mise en contact avec l'agent ;

e) la perméabilisation des cellules dans chaque aliquote d'essai et aliquote témoin ;

f) la mise en contact des cellules perméabilisées avec un peptide du domaine BH3 pro-apoptotique ;

g) la détermination d'une valeur du pourcentage de la MOMP induite par le peptide du domaine BH3 dans les cellules dans chaque aliquote d'essai ;

h) la détermination d'une valeur du pourcentage de la MOMP induite par le peptide du domaine BH3 dans les cellules dans chaque aliquote témoin ; et

i) la détermination d'une valeur du pourcentage d'amorçage delta pour chaque aliquote d'essai, ledit pourcentage d'amorçage delta étant la différence entre la valeur du pourcentage de la MOMP déterminée en (g) et la valeur du pourcentage de la MOMP déterminée en (h),

un pourcentage d'amorçage delta supérieur à 20 pour cent indiquant que l'agent est toxique pour le tissu sur lequel les cellules ont été prélevées.

10. Méthode selon la revendication 9, comprenant en outre la réalisation d'essais de toxicité *in vivo* avec l'agent chez un animal non humain ou d'essais de toxicité *in vitro* avec l'agent si le pourcentage d'amorçage delta est inférieur ou égal à 20 pour cent, inférieur ou égal à 18 pour cent, inférieur ou égal à 16 pour cent, inférieur ou égal à 14 pour cent, inférieur ou égal à 12 pour cent, inférieur ou égal à 10 pour cent, inférieur ou égal à 8 pour cent, inférieur ou égal à 6 pour cent, inférieur ou égal à 4 pour cent, inférieur ou égal à 2 pour cent, ou inférieur ou égal à 1 pour cent.

11. Méthode selon la revendication 9 ou 10 :

i) lesdites cellules non cancéreuses étant des cellules saines ; et/ou

ii) lesdites cellules étant des cellules de mammifères ; et/ou

iii) lesdites cellules étant dérivées d'échantillons prélevés par biopsie ou autopsie d'un animal ; et/ou

iv) lesdites cellules de chaque aliquote d'essai étant mises en contact avec l'agent pendant 24 heures ou moins ; et/ou

v) lesdites cellules perméabilisées étant mises en contact avec le peptide du domaine BH3 pro-apoptotique pendant 3 heures ou moins ; et/ou

vi) lesdites cellules étant perméabilisées avec de la digitonine ou de la saponine.

**12.** Méthode selon l'une quelconque des revendications 9 à 11, ledit pourcentage de la MOMP induite par le peptide du domaine BH3 étant déterminé par une ou plusieurs parmi :

i) la mise en contact des cellules avec un colorant potentiométrique et la mesure de l'émission dudit colorant potentiométrique, ledit colorant potentiométrique étant choisi dans le groupe constitué par l'iodure de 5,5',6,6'-tétrachloro-1,1',3,3'-tétraéthylbenzimidazolylcarbocyanine (JC-1), la dihydrorhodamine 123, l'ester méthylique de tétraméthylrhodamine (TMRM) ou l'ester éthylique de tétraméthylrhodamine (TMRE) ;

ii) la mesure de la libération d'une molécule provenant de l'espace intermembranaire mitochondrial, ladite molécule provenant de l'espace intermembranaire mitochondrial étant choisie dans le groupe constitué par le cytochrome c, le SMAC/Diablo, l'Omi, l'adénylate kinase-2 ou le facteur inducteur de l'apoptose ; et

iii) la mesure de la rétention d'une molécule provenant de l'espace intermembranaire mitochondrial, ladite molécule provenant de l'espace intermembranaire mitochondrial étant choisie dans le groupe constitué par le cytochrome c, le SMAC/Diablo, l'Omi, l'adénylate kinase-2 ou le facteur inducteur de l'apoptose.

**13.** Méthode selon l'une quelconque des revendications 9 à 12 :

i) ledit peptide du domaine BH3 étant dérivé du domaine BH3 d'un BID, d'un BIM, d'un BAD, d'une NOXA, d'un PUMA, d'un BMF ou d'un polypeptide HRK ; et/ou

ii) ledit peptide du domaine BH3 étant choisi dans le groupe constitué par les SEQ ID n° : 1 à 16 ; et/ou

iii) ledit agent comprenant un ou plusieurs composés ; et/ou

iv) ledit agent étant un agent anticancéreux ; et/ou

v) ledit agent étant un agent chimiothérapeutique ; et/ou

vi) ledit agent étant une petite molécule organique, une petite molécule inorganique, un peptide, une protéine, un analogue de protéine, une enzyme, un acide nucléique, un analogue d'acide nucléique, un anticorps, un antigène, une hormone, un lipide, un polysaccharide, un facteur de croissance, un virus, une cellule, un agent bioactif, un agent pharmaceutique, ou une combinaison ou un promédicament de ceux-ci ; et/ou

vii) ledit agent étant un gaz, des particules fines, un rayonnement, un rayonnement électromagnétique ou un aérosol.

**14.** Méthode de prédiction de la toxicité d'un agent, ladite méthode comprenant :

a) la fourniture d'un ou plusieurs échantillons de cellules, chaque échantillon de cellules comprenant des cellules non cancéreuses préalablement prélevées sur un tissu provenant d'un animal non humain qui a été mis en contact avec une dose sublétale d'un agent puis a été sacrifié ;

b) la fourniture d'une aliquote d'essai de chacun desdits un ou plusieurs échantillons de cellules ;

c) la mise en contact séparément de chaque aliquote d'essai avec l'agent ;

d) la fourniture d'une aliquote témoin de chacun desdits un ou plusieurs échantillons de cellules, ladite aliquote témoin n'étant pas mise en contact avec l'agent ;

e) la perméabilisation des cellules dans chaque aliquote ;

f) la mise en contact des cellules perméabilisées avec un peptide du domaine BH3 pro-apoptotique ;

g) la déterminer d'une valeur du pourcentage de la MOMP induite par le peptide du domaine BH3 dans les cellules dans l'aliquote d'essai ;

h) la détermination d'une valeur du pourcentage de la MOMP induite par le peptide du domaine BH3 dans les cellules dans l'aliquote témoin ; et

i) la détermination d'une valeur du pourcentage d'amorçage delta pour l'aliquote d'essai, ledit pourcentage d'amorçage delta étant la différence entre la valeur du pourcentage de la MOMP déterminée en (g) et la valeur du pourcentage de la MOMP déterminée en (h),

un pourcentage d'amorçage delta supérieur à 20 pour cent indiquant que l'agent est toxique pour le tissu de l'animal non humain sur lequel les cellules ont été prélevées.

**15.** Méthode selon la revendication 14 :

i) ledit animal non humain qui a été mis en contact avec une dose sublétale d'un agent étant un mammifère, ledit mammifère étant éventuellement un primate non humain ou un rongeur ; et/ou

ii) ledit animal non humain ayant été mis en contact avec l'agent par injection, inhalation, administration topique ou administration orale ; et/ou

iii) ledit animal non humain étant en contact avec l'agent pendant 24 heures ou moins ; et/ou

iv) lesdites cellules perméabilisées étant mises en contact avec le peptide du domaine BH3 pro-apoptotique pendant 3 heures ou moins ; et/ou

v) lesdites cellules étant perméabilisées à la digitonine ou à la saponine ; et/ou

vi) ledit peptide du domaine BH3 étant dérivé du domaine BH3 d'un BID, d'un BIM, d'un BAD, d'une NOXA, d'un PUMA, d'un BMF ou d'un polypeptide HRK ; et/ou

vii) ledit peptide du domaine BH3 étant choisi dans le groupe constitué par les SEQ ID n° : 1 à 16 ; et/ou

viii) ledit agent comprenant un ou plusieurs composés ; et/ou

ix) ledit agent étant un agent anticancéreux ; et/ou

x) ledit agent étant un agent chimiothérapeutique ; et/ou

xi) ledit agent étant une petite molécule organique, une petite molécule inorganique, un peptide, une protéine, un analogue de protéine, une enzyme, un acide nucléique, un analogue d'acide nucléique, un anticorps, un antigène, une hormone, un lipide, un polysaccharide, un facteur de croissance, un virus, une cellule, un agent bioactif, un agent pharmaceutique, ou une combinaison ou un promédicament de ceux-ci ; et/ou

xii) ledit agent étant un gaz, des particules fines, un rayonnement, un rayonnement électromagnétique ou un aérosol.

FIG. 1

FIG. 1 cont.

FIG. 2

FIG. 3

FIG. 4

FIG. 4 cont.

FIG. 5

FIG. 5 cont.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014047342 A
- WO 2015010094 A

### Non-patent literature cited in the description

- **BARRETINA et al.** The Cancer Cell Line Encyclopedia enables predictive modelling of anticancer drug sensitivity. *Nature,* 28 March 2012, vol. 483 (7391), 603-7
- **PETRUCCELLI ; CHEN ; NANDRAM.** Applied Statistics for Engineers and Scientists. 1999
- **RYAN, J. ; LETAI, A.** *Methods,* 2013, vol. 61 (2), 156-164
- **FRIEDMAN, A. ; LETAI, A. ; FISHER, D. ; FLAHERTY, K.** *Nature Reviews Cancer,* 2015, vol. 15, 747-756
- **BROCK et al.** *Sci Transl Med,* 2014, vol. 6, 217ra2
- **SAMSON et al.** *Endocrinology,* 1996, vol. 137, 5182-5185
- **DEGRADO.** *Adv Protein Chem,* 1988, vol. 39, 51-124